# EUROPEAN PATENT SPECIFICATION

(11) **EP 3 952 752 B1**
(45) Date of publication and mention of the grant of the patent: **20.11.2024**
(21) Application number: 20787461.1
(22) Date of filing: 07.04.2020
(51) Int. Cl.: A61B 17/34, A61B 17/32, A61B 34/30, A61B 34/32, A61B 17/00

(54) **SELF-STRAIGHTENING NEEDLE ASSEMBLY**
SELBSTBEGRADIGENDE NADELANORDNUNG
ENSEMBLE AIGUILLE À AUTO-REDRESSEMENT

(30) Priority: 10.04.2019 IL 26596319
(43) Date of publication of application: 16.02.2022
(73) Proprietor: Tamar Robotics Ltd, 3006500 Kibbutz Yagur (IL)
(72) Inventor: SHTENDEL, Tom, 3603220 Kiryat-Tivon (IL); ZISO, Hadas, 3605421 Kiryat-Tivon (IL); HASSIDOV, Noam, 2521300 Doar-Na Oshrat (IL)
(74) Representative: ip21 Ltd
(86) International application number: PCT/IL2020/050427
(87) International publication number: WO 2020/208634

(56) References cited:
- EP-A2- 2 521 501
- WO-A1-99/58055
- US-A- 5 788 713
- US-A1- 2013 158 578
- US-A1- 2013 197 306
- US-A1- 2016 346 513

## Description

### FIELD AND BACKGROUND OF THE INVENTION

The present invention, in some embodiments and examples thereof, relates to the field of robotic systems for use in minimally invasive surgical and diagnostic procedures, and more particularly to use in performing intracranial therapies in the field of neurosurgery.

In US Patent Application Publication No. 2009/0048610 to G. Tolkowsky et al., for "Medical Probe Introducer" there is described a hand operated mechanical system for inserting a probe into the subject's cranial tissue, in which the off-axis target of the probe or the treatment is reached, either by a use of a straight needle with an angled treatment outlet tip, or by use of a needle constructed of a shape memory alloy, which bends on exiting its outer cannula in order for the tip to reach the treatment area.

In US Patent Application Publication No. 2004/0059260 to C. L. Truwit, for "Method and Device for Deflecting a Probe", there is described a method and device for inserting a flexible needle down a cannula having an off-axis exit aperture, such that as it exits from the cannula, the needle can access a region off-axis from the region to which the straight cannula can reach.

In International Patent Application Publication No. WO2012/095845, for "Robot For Minimally Invasive Neurosurgery", having a common inventor with the present application, there is described the use of a robotic device in combination with a optically detectable tumor marker for real-time detection and treatment of the tumor, using a cannulated needle for insertion into the cranial tissue, with a flexible inner needle disposed coaxially within the cannulated needle and able to exit the cannulated needle at angles up to 90° to the axis of the cannulated needle. Rotation of the cannulated needle enables access to tissue disposed at any angle azimuthal to the cannulated needle axis. The flexible needle is contained within an outer cannula, the flexible needle being controlled by the robot to provide motion into and out of a non-axial aperture in the distal part of the cannula. The needle has a structure that enables it to exit the non-axial aperture and to continue moving out of the aperture to the target region without losing its mechanical properties.

In US published Patent Application No. US 2014/0025088, having a common inventor with the present application, there are described a number of possible structures having such mechanical properties, including a chain of magnetized segments, flattened tube structures, and tensegrity structures. The coordinated control of the insertion motion of the cannula and the flexible needle and rotation of the rotatable element enables the needle to access a target region of the brain.

U.S. Patent Publication No. 2013/0158578 A1 relates to "Neurosurgical devices including or used with cannulas or catheters and associated systems and methods are disclosed herein. The neurosurgical devices can include, for example, a cannula having a main portion and an angle-forming member proximate a distal end of the main portion. The angle-forming member can be configured to transition from a substantially straight configuration while the cannula is advanced through tissue along a substantially straight first portion of a path to an angled configuration when the angle-forming member reaches an end of the substantially straight first portion of the path."

U.S. Patent No. 5,788,713 relates to "A device for the percutaneous localization of a foreign object in a body has a base collar mount and one or more movable collar mounts all located on at least one perpendicular guide rail. The base collar mount has a guide-tube cannula affixed to it. A series of tubings and wire lengths extend through the guide-tube cannula as the movable collar mounts are advanced. As the tubings and wire are exposed from the guide-tube cannula, they assume their native curvature thus forming a compound trajectory for the insertion of a foreign object in a body."

U.S. Patent Publication No. 2016/0346513 A1 relates to "...the ability [of needle-sized surgical tools] to navigate around sharp corners to manipulate or visualize tissue. A needle-sized bendable joint design that grants this ability. It can be easily interfaced with manual tools or concentric tube robots and is straightforward and inexpensive to manufacture. The bendable joint includes of a nitinol tube with several asymmetric cutouts, actuated by a tendon."

### SUMMARY

Aspects of the present invention are set out by the claims. Further aspects are also described. The invention relates to a needle assembly according to claim 1. Methods of treatment or surgery are not claimed.

According to some embodiments of the present disclosure, the needle assembly further includes retaining devices positioned at intermediate locations along the slotted wall, adapted to thereby maintain the wire in position along the slotted wall.

According to some embodiments of the present disclosure, any of the first cannula, the second cannula, and the inner element are robotically controlled to provide at least one of extension and rotational motion.

According to some embodiments of the present disclosure, the needle assembly is robotically controlled according to a pre-surgical plan, such that a tip of the inner element can reach a target region.

According to some embodiments of the present disclosure, extension of the second cannula through the tissue is adapted to trace a track set by the known angle of curvature of the second cannula, such that damage to the tissue is minimized.

According to some comparative examples of the present disclosure, the needle assembly is adapted to carry a surgical tool, the surgical tool being one of a suction, a drill, a cautery, a needle, a camera, and a tissue ablator.

According to some comparative examples of the present disclosure, a controller is configured to determine the position of the end region of the second cannula.

According to some embodiments of the present disclosure, an angle of the bending is dependent on the ratio between the width of the slots and the width of the wall between the slots.

According to some embodiments of the present disclosure, the position of the distal end of the second cannula is determined by the extent of the extension of the second cannula out of the first cannula.

According to some comparative examples of the present disclosure, the wire is adapted to provide the slotted second cannula with increased stiffness, such that the distal end has increased resistance to displacement when the second cannula is exposed to lateral forces.

According to some comparative examples of the present disclosure, a controller is configured to determine the position of a tip of the self-straightening inner element.

According to some comparative examples of the present disclosure, the self-straightening inner element includes at least one of a super-elastic tube; a memory alloy tube; a slotted tube; a tightly coiled memory alloy spring; circular links held together by pulling cables preloaded by springs; a notched inner cannula with a cable connected via a loaded spring to tension the inner cannula; and a solid element carrying or including a surgical tool, a fiber optic cable, and a camera.

According to an aspect of some comparative examples of the present disclosure, there is provided a needle assembly for extension through tissue in a surgical procedure, including: a stiff, straight outer first cannula; a second cannula at least whose distal end region has a naturally curved shape and whose stiffness and diameter is less than that of the outer cannula, such that the second cannula is adapted to be passed in a straightened form through the stiff straight outer first cannula; and a self-straightening inner element disposed within and adapted to pass through the second cannula, the inner element having a stiffness less than that of the second cannula such that it can pass through the second cannula whether the second cannula is straight or curved, but emerges in its self-straightened form.

According to some comparative examples of the present disclosure, the needle assembly further includes a robotic controller adapted to control at least one of extension and rotation of at least one of the first cannula, second cannula, and the inner element, such that the position of a distal end region of the inner element is known.

According to some comparative examples of the present disclosure, extension of the second cannula through the tissue is adapted to trace a path set by the naturally curved shape of the second cannula, such that damage to the tissue is minimized.

According to some comparative examples of the present disclosure, the needle assembly is adapted to carry a surgical tool, the surgical tool being one of a suction, a drill, a cautery, a needle, a camera, and a tissue ablator.

According to some comparative examples of the present disclosure, the naturally curved shape and the stiffness of the second cannula are such that the position of the distal end of the second cannula is known at any point during passage of second cannula out of the first cannula.

According to some comparative examples of the present disclosure, the second cannula includes a biocompatible material having at least one of superelastic properties and a shape memory.

According to an aspect of some comparative examples of the present disclosure, there is provided a system for performing a surgical procedure according to a pre-surgical plan at a target region of a subject's brain, including: (a) a robot having a controller, the robot adapted to execute the surgical procedure via the controller according to the pre-surgical plan; (b) a needle assembly adapted to be controlled by the robot, including: a stiff, straight outer cannula; a middle cannula disposed within the outer cannula, and having a naturally curved shape and a stiffness less than that of the outer cannula, such that the middle cannula can be passed through the straight outer cannula; and a self-straightening inner element disposed within the middle cannula, the inner element having a stiffness less than that of the naturally curved middle cannula such that is adapted to pass through the middle cannula and adopt its straight form after exiting the second cannula; wherein robotically-controlled insertion of at least one of the outer cannula, the middle cannula, and the inner element is adapted to generate a unique specified path to the target region which minimizes damage to brain tissue.

According to an aspect of some comparative examples of the present disclosure, there is provided a method for performing a surgical procedure on a target region of a subject's brain according to a pre-surgical plan, including: (a) planning a path to the target region following a tip of a tool to be inserted to perform the surgical procedure, the path configured to avoid damage-sensitive regions of the brain; (b) inserting extendable elements under robotic control into the subject's brain along the path, a distal region of the extendable elements having a single, known position at any point of extension, the distal region carrying the tool; and (c) executing the pre-surgical plan using the tool such that the position of the tool is known to the robot throughout the surgical procedure.

According to some comparative examples of the present disclosure, the target region and the surgical procedure are in any organ or tissue of the human body.

According to an aspect of some comparative examples of the present disclosure, there is provided a needle assembly for guided extension through tissue in a surgical procedure, including: a stiff, straight outer first cannula; a second cannula adapted to be passed through and extended out of the stiff straight outer first cannula; a wire disposed within the second cannula, along a wall of the second cannula and attached to a distal end region of the second cannula, such that, when the wire is under tension, it is adapted to bend the second cannula; and a self-straightening inner element disposed within the second cannula, the self-straightening inner element having a flexibility such that it passes through the second cannula even when the second cannula is bent, but adopts its straight form after exiting the second cannula.

According to some comparative examples of the present disclosure, the second cannula has lateral slots along at least part of its length; and wherein the wire under tension bends the second cannula until adjacent sides of the slots in the extended portion of the second cannula abut each other, the abutting of the sides of the slots defining a known angle of curvature of the second cannula.

According to some embodiments of the present disclosure, the slots extend across a majority of the circumferential wall of the cannula.

Unless otherwise defined, all technical and/or scientific terms used herein have the same meaning as commonly understood by one of ordinary skill in the art to which the present disclosure pertains. Although methods and materials similar or equivalent to those described herein can be used in the practice or testing of comparative examples of the present disclosure, exemplary methods and/or materials are described below. In case of conflict, the patent specification, including definitions,
will control. In addition, the materials, methods, and examples are illustrative only and are not intended to be necessarily limiting.

As will be appreciated by one skilled in the art, aspects and examples of the present disclosure may be embodied as a system, method or computer program product. Accordingly, aspects of the present disclosure may take the form of an entirely hardware example, an entirely software example (including firmware, resident software, microcode, *etc.)* or an example combining software and hardware aspects that may all generally be referred to herein as a "circuit," "module" or "system" (e.g., a method may be implemented using "computer circuitry"). Furthermore, some comparative examples of the present disclosure may take the form of a computer program product embodied in one or more computer readable medium(s) having computer readable program code embodied thereon. Implementation of the method and/or system of some comparative examples of the present disclosure can involve performing and/or completing selected tasks manually, automatically, or a combination thereof. Moreover, according to actual instrumentation and equipment of some comparative examples of the method and/or system of the present disclosure, several selected tasks could be implemented by hardware, by software or by firmware and/or by a combination thereof, e.g., using an operating system.

For example, hardware for performing selected tasks according to some examples of the present disclosure could be implemented as a chip or a circuit. As software, selected tasks according to some examples of the present disclosure could be implemented as a plurality of software instructions being executed by a computer using any suitable operating system. In some comparative examples of the present disclosure, one or more tasks performed in method and/or by system are performed by a data processor (also referred to herein as a "digital processor", in reference to data processors which operate using groups of digital bits), such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well. Any of these implementations are referred to herein more generally as instances of computer circuitry.

Any combination of one or more computer readable medium(s) may be utilized for some examples of the present disclosure. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device. A computer readable storage medium may also contain or store information for use by such a program, for example, data structured in the way it is recorded by the computer readable storage medium so that a computer program can access it as, for example, one or more tables, lists, arrays, data trees, and/or another data structure. Herein a computer readable storage medium which records data in a form retrievable as groups of digital bits is also referred to as a digital memory. It should be understood that a computer readable storage medium, in some comparative examples, is optionally also used as a computer writable storage medium, in the case of a computer readable storage medium which is not read-only in nature, and/or in a read-only state.

Herein, a data processor is said to be "configured" to perform data processing actions insofar as it is coupled to a computer readable memory to receive instructions and/or data therefrom, process them, and/or store processing results in the same or another computer readable storage memory. The processing performed (optionally on the data) is specified by the instructions. The act of processing may be referred to additionally or alternatively by one or more other terms; for example: comparing, estimating, determining, calculating, identifying, associating, storing, analyzing, selecting, and/or transforming. For example, in some comparative examples, a digital processor receives instructions and data from a digital memory, processes the data according to the instructions, and/or stores processing results in the digital memory. In some comparative examples, "providing" processing results comprises one or more of transmitting, storing and/or presenting processing results. Presenting optionally comprises showing on a display, indicating by sound, printing on a printout, or otherwise giving results in a form accessible to human sensory capabilities.

A computer readable signal medium may include a propagated data signal with computer readable program code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, *etc.,* or any suitable combination of the foregoing.

Computer program code for carrying out operations for some comparative examples of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some comparative examples of the present disclosure may be described below with reference to flowchart illustrations and/or block diagrams of methods, apparatus (systems) and computer program products according to examples of the present disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

Some of the methods described herein are generally designed only for use by a computer, and may not be feasible or practical for performing purely manually, by a human expert. A human expert who wanted to manually perform similar tasks, such as a medical practitioner, might be expected to use completely different methods, e.g., making use of expert knowledge and/or the pattern recognition capabilities of the human brain, which would be vastly more efficient than manually going through the steps of the methods described herein.

### BRIEF DESCRIPTION OF THE SEVERAL VIEWS OF THE DRAWINGS

Some embodiments of the present disclosure are herein described, by way of example only, with reference to the accompanying drawings. With specific reference now to the drawings in detail, it is stressed that the particulars shown are by way of example, and for purposes of illustrative discussion of embodiments and examples of the present disclosure. In this regard, the description taken with the drawings makes apparent to those skilled in the art how embodiments of the present disclosure may be practiced.

In the drawings:
FIGS. 1A-1D show a prior art endoscope with two angulation pulling wires oriented on either side of the flexible scope, illustrating how either of two positions could be defined by a given movement of the pulling wires, according to some examples of the present disclosure;
FIGS. 2A-2C show the effects of an imprecise steering mechanism in a prior art steering segment track, according to some examples of the present disclosure;
FIGS. 3A-3C diagram the concept of the current invention, showing how an exact position of the tip is defined by a tensioned inner cannula inside of an outer one, according to some examples of the present disclosure;
FIGS. 4A-4H illustrate the three cannulas of the currently disclosed steerable and self-straightening needle and the manner in which the mechanism operates, according to some examples of the present disclosure;
FIGS. 5-01 to 5-17 illustrates one of the manner in which a steerable needle operates to remove a tumor or other lesion, according to some examples of the present disclosure;
FIG. 6 illustrates the manner in which the steerable needle is controlled by a robot and controller system, according to some examples of the present disclosure;
FIGS. 7A-7I illustrate the design of the middle cannula, allowing both flexibility and full control of the steering segment, according to some examples of the present disclosure;
FIGS. 8A-8C show how the middle cannula moves out of the stiff outer cannula at a precisely defined angle, according to some examples of the present disclosure;
FIGS. 9A-9C show extension of the self-straightening inner cannula from within the bent middle cannula, according to some examples of the present disclosure; and
FIGS. 10A-10I illustrate several means of making a self-straightening inner cannula of specific flexibility and having a bendable steering segment, according to some examples of the present disclosure.

### DESCRIPTION OF SPECIFIC EXAMPLES AND EMBODIMENTS OF THE

### INVENTION

The present invention, in some examples and embodiments thereof, relates to the field of robotic systems for use in minimally invasive surgical and diagnostic procedures, and more particularly to use in performing intracranial therapies in the field of neurosurgery.

### Introduction

A shape memory needle traversing a curved path upon exiting its outer cannula potentially introduces levels of inaccuracy which may be problematic for treatment of cranial tissue. Unlike a straight needle that may have high rigidity, tip of the needle may not proceed in an arcuate path to its intended target, for two reasons. First, the curved needle trajectory may be inaccurate or imprecise. Second, side forces applied to the needle during passage, such as varying tissue densities, may deflect the needle from its planned path. Resulting deflection in the needle path may cause the tip of the needle to miss the target. Micrometer-based systems may be slow to operate and/or dependent on settings being adjusted by the surgeon to achieve reasonable accuracy of insertion.

In some examples and embodiments of the present disclosure, mechanical properties of the materials of the flexible needle, and/or the shape of a flexible needle are specifically adjusted to enhance needle aiming accuracy.

Two conflicting requirements may apply to a neurosurgical device of the type described in this disclosure. In the first place, the outer diameter of the external cannular needle is preferably as small as possible in order to reduce trauma to healthy brain tissue to a minimum. The exit aperture path should be contained within the outer cannula external diameter limits, as otherwise, it would interfere with the smooth initial insertion of the outer cannula into the brain tissue.

Furthermore (and in the second place), after achieving this bend, the flexible needle should be sufficiently strong to be capable of penetrating brain tissue, to withstand lateral forces due to anisotropic nature of the brain tissue, to deploy in a straight line to reach the desired target region with the accuracy required, and to support any axial force which may be required for it to perform its surgical or therapeutic function, or which may be operative on it while moving through the brain tissue. These conflicting requirements mandate novel and inventive configurations of the system and of the flexible needle.

In cranial surgery, a preoperative treatment plan, based on any preoperative imaging modality, such as MRI or CT, is optionally used in order to determine the three-dimensional location and extent of any tissue anomaly, such as a tumorous growth, within the patient's brain tissue. Preoperative images may then be used to determine the surgical procedure to be performed on the growth.

In some examples and embodiments, motion of the cannulated needle and the flexible inner needle are robotically controlled, with the robotic coordinate system registered to the patient's skull and hence to the details on the preoperative images.

Potential problems in targeting arise in relation to brain shift effects, leakage of cerebrospinal fluid (CSF), and/or resulting from treatment of the brain matter or the insertion of the robotic probe into the brain tissue. These potential problems make systems in which treatment location is based solely on the expected position of the previously imaged targeted tissue, a risky or even dangerous procedure, since it is not known where the treatment is actually being performed.

Accordingly, the inventors have identified a need for a robotically controlled surgical system which takes into account motion of the tissue being operated on, which enables the treatment to be performed safely only on the diseased tissue which it is desired to treat while leaving any healthy tissue intact.

### Overview

An aspect of some examples and embodiments of the present disclosure relates to systems for a needle assembly to be inserted and steered within the brain, body tissue, or cavity, for the purpose of performing a surgical procedure such as tissue cutting, ablation, or removal. The needle assembly, adapted for either or both of diagnostic and therapeutic purposes, comprises a set of robotically controlled and motorized concentric cannulas, such that the position of the tip of the needle assembly is exactly known by means of the robotic control system.

More particularly, in some examples and embodiments, the needle assembly is designed so that its flexible inner tip is minimally susceptible to displacement from its intended path by forces applied to it from any direction as it is advanced through the tissue. The needle assembly follows the track set by the inner cannula tip, thereby causing minimal damage to the surrounding brain tissue. Thus, both the position of the needle tip and the track of the needle insertion tube are pre-defined and known, enabling the operator to avoid critical features that should not be contacted by the needle during insertion or surgical manipulation.

In some examples and embodiments, the components of this novel steerable needle comprise a series of three concentric cannulas, having the following characteristics. The outer cannula is stiff and straight, and may be constructed, for example, of stainless steel. The middle cannula has a distal steering segment that may be pre-bent to have a natural curvature. The middle cannula is flexible enough to be straightened when disposed inside the outer cannula, but retains a memory of its shape and returns to its natural curved shape when extended outward from the confines of the outer cannula. The shape memory may result from the material properties of a shape memory alloy, such as nitinol, from a curve-shaped plastic spring of defined stiffness, from a metal tube with partial cuts, or any mechanical solution resulting in a cannula with the desired degree of stiffness and having a shape memory. The inner cannula is comprised of a more flexible material than the middle cannula and is configured to be straight except when situated within the curved steering segment of the stiffer middle cannula. The middle and inner cannulas are extended, for example, by external motors that can cause extension or rotation, allowing the tip of the inner cannula to reach various orientations at a range of depths. The ability of the extendable middle and inner cannulas to be inserted at various angles and depths allows the device to reach asymmetrical lesions by altering the extension as a function of the angle of the middle cannula, and extension of the straight inner cannula. The tip of the inner cannula can be configured to be either a hollow needle, or to hold a surgical tool.

Some potential advantages of the currently disclosed steerable needle assembly are now listed. The trajectory of the current steerable needle can be well-defined along its whole length, such that the extendable tip traces a trajectory that is then followed by the subsequent segments of the cannula. Thus, the width of the trajectory is limited to approach the diameter of the needle assembly itself. The tip then follows a single path, rather than traversing additional areas during needle insertion.

Another potential advantage of the present invention is that the position of the tip of the needle is known to a registration system of a robotic controller. This robotic control system allows the surgeon to program an operation according to lesion identification on pre-surgical images and to manipulate the needle or other surgical tool with precision, avoiding critical areas that could be damaged by the needle.

A further potential advantage of the steerable needle assembly is that it is designed to have a middle cannula that is bendable and yet, using an internal, tensioned wire, can be kept rigid once it extends from within a straight outer cannula and adopts its bent or curved state. This design ensures that the tip is not deflected by side forces such as those encountered when traversing organs with variable tissue densities or structures. In one exemplary implementation, the design of the middle cannula may be a tube with regularly spaced partial slots along the distal steering segment, weakening the stiffness of the cannula in that segment and allowing it to be curved or bent in a pre-defined manner. This design makes use of a wire running along the cut length of the cannula, and attached to a spring at the distal end of the cannula, such that the wire is under constant tension. When the middle cannula extends from the straight, stiff outer cannula, the tension of the external linear spring on the wire causes the slotted portion of the steering segment to curve such that it closes the slots. Because the slots and the intervening segments of the middle cannula have defined widths, the middle cannula curves at a known angle. When the middle cannula is fully contained within the stiff outer cannula, the middle cannula is straight and the spring is under greatest tension. As the tip and steering segment of the middle cannula are extended forward from the outer cannula, the steering segment curves as the spring pulls on the wire. The stiffness of the spring attached to the wire is calculated with a built-in pre-stress/pre-load factor, such that the spring may exert force on the wire several times that needed to maintain the curvature of the steering segment during extension through tissue, even when the tip of the middle cannula is exposed to forces that would tend to deflect the tip from its planned path toward a target. This design thus allows both flexibility of the steering segment and yet a stiffness that prevents the tip from deviating from its planned path.

The present needle assembly design allows the surgeon to precisely perform delicate operations to incise, ablate, and/or remove diseased tissue or blood hemorrhage from the brain or other solid and semi-solid organs. Advantages of the presently disclosed needle assembly are several. It minimizes collateral tissue damage, thus being suitable for minimally invasive procedures. Also, the tip of the inner cannula can be determined and followed with precision throughout manipulation of the needle assembly, due to the selective mechanical properties of the three cannulas.

It is to be understood that, while the illustrations given in this disclosure relate primarily to the brain, such a steering mechanism is not limited to use in intracranial procedures but is also suitable for use in treatment of other organs in the body. The needle assembly is designed to be single-use; however, it is possible to render the assembly capable of multi-use design.

One exemplary implementation involves a needle assembly for guided extension through tissue in a surgical procedure, having three concentric elements, the first being a stiff, straight outer cannula; within the outer cannula is a second cannula, adapted to be passed through and extended out of said stiff straight outer first cannula. The second cannula has lateral slots along at least part of its length extending across a majority of the circumferential wall of the second cannula. Within the second cannula is disposed a wire along the slotted wall and attached to a distal end region of said second cannula, such that, when the wire is under tension, the second cannula bends until adjacent sides of the slots in the extended portion of the cannula abut each other. This configuration in which the sides of the slots are in contact, defines a known angle of curvature of the second cannula. The third concentric part is a self-straightening inner element disposed within the second cannula. This inner element has a flexibility such that it passes through the second cannula even when the second cannula is bent, but adopts its straight form after exiting the second cannula.

In further exemplary implementations, the wire within the second cannula may be tensioned by means of a spring connected between the wire and an anchor point on a proximal point of the second cannula. The wire may further be adapted to provide said slotted second cannula with increased stiffness, such that said distal end has increased resistance to displacement when said second cannula is exposed to lateral forces. The second cannula may further comprise retaining devices positioned at intermediate locations along its slotted wall, adapted to thereby maintain the wire in position along the slotted wall. The angle of bending of the second cannula may be dependent on the ratio between the width of the slots and the width of the wall between the slots, and the position of the distal end of the second cannula may be determined by the extent of its extension out of said first cannula.

Any of the concentric elements, i.e., the first cannula, the second cannula, and the inner element may be robotically controlled to provide extension and/or rotational motion. The robotic control may be according to a pre-surgical plan, such that the inner element tip can reach a target region. The inner element may be adapted to comprise or to carry a surgical tool, the surgical tool being one of a suction, a drill, a cautery, a needle, a camera, and a tissue ablator. The second cannula may comprise a curved distal end region, such that extension of the second cannula from within the outer cannula traces a track set by the natural curvature of the second cannula in order to avoid or minimize tissue damage; this track may be programmed by a controller configured to determine the position of at least one of the end region of the second cannula and the position of the distal tip of the self-straightening inner element.

The self-straightening inner element of the needle assembly may comprise at least one of a super-elastic tube; a memory alloy tube; a slotted tube; a tightly coiled memory alloy spring; circular links held together by pulling cables preloaded by springs; a notched inner cannula with a cable connected via a loaded spring to tension the inner cannula; and a solid element carrying or comprising a surgical tool, a fiber optic cable, and a camera.

In another exemplary implementation, the needle assembly for extension through tissue in a surgical procedure may comprise a stiff, straight outer first cannula; a second cannula at least whose distal end region has a naturally curved shape and whose stiffness and diameter is less than that of the outer cannula, such that the second cannula is adapted to be passed in a straightened form through the stiff straight outer first cannula; and a self-straightening inner element disposed within and adapted to pass through said second cannula, said inner element having a stiffness less than that of the second cannula such that it can pass through said second cannula whether said second cannula is straight or curved, but emerges in its self-straightened form.

Such an assembly may further comprise a robotic controller adapted to control at least one of extension and rotation of at least one of the three: first cannula, second cannula, and said inner element, such that the position of a distal end region of the inner element is known. Extension of the second cannula through the tissue traces a path set by the natural curve of the second cannula, such that damage to said tissue is minimized. The naturally curved shape and said stiffness of said second cannula are such that the position of said distal end of said second cannula is known at any point during passage of the second cannula out of the first cannula. The second cannula may comprise a biocompatible material having at least one of superelastic properties and a shape memory. The needle assembly may be adapted to carry a surgical tool, such as a suction, a drill, a cautery, a needle, a camera, or a tissue ablator.

An exemplary system using the needle assembly for performing a surgical procedure according to a pre-surgical plan at a target region of a subject's brain may comprise the following: a robot adapted to execute the surgical procedure via a controller according to said pre-surgical plan; a needle assembly adapted to be controlled by the robot, comprising: a stiff, straight outer cannula; a middle cannula disposed within the outer cannula, and having a naturally curved shape and a stiffness less than that of the outer cannula, such that the middle cannula can be passed through the straight outer cannula; and a self-straightening inner element disposed within the middle cannula, the inner element having a stiffness less than that of the naturally curved middle cannula such that is adapted to pass through the middle cannula and adopt its straight form after exiting the second cannula; wherein robotically-controlled insertion of at least one of the outer cannula, the middle cannula, and the inner element is adapted to generate a single, specified path to the target region which minimizes damage to brain tissue.

The present disclosure, not belonging to the invention, further reveals a method for performing a surgical procedure on a target region of a subject's brain or any other organ or tissue, according to a pre-surgical plan, comprising: (a) planning a path to said target region following a tip of a tool to be inserted to perform said surgical procedure, the path configured to avoid damage-sensitive regions of the brain; (b) inserting extendable elements under robotic control into the subject's brain along the path, a distal region of the extendable elements having a single, known position at any point of extension and carrying the tool; and (c) executing the pre-surgical plan using the tool such that the position of the tool is known to the robot throughout the surgical procedure.

Throughout this application, the terms `angle of bending' or `angle of curvature' are used, and in some cases claimed, to describe the extent of bending or curving of a cannula or other component of the needle assembly. It is to be understood that any reference to these or similar terms is meant to refer to the level of curvature as scientifically defined by `radius of curvature' for an arc of a circle or `degree of curvature' for more general shapes; other mathematical terms may be applicable in specific instances.

Before explaining at least one embodiment of the present disclosure in detail, it is to be understood that the present disclosure is not necessarily limited in its application to the details of construction and the arrangement of the components and/or methods set forth in the following description and/or illustrated in the drawings. Features described in the current disclosure, including features of the invention, are capable of other embodiments or of being practiced or carried out in various ways.

### Embodiments and Examples

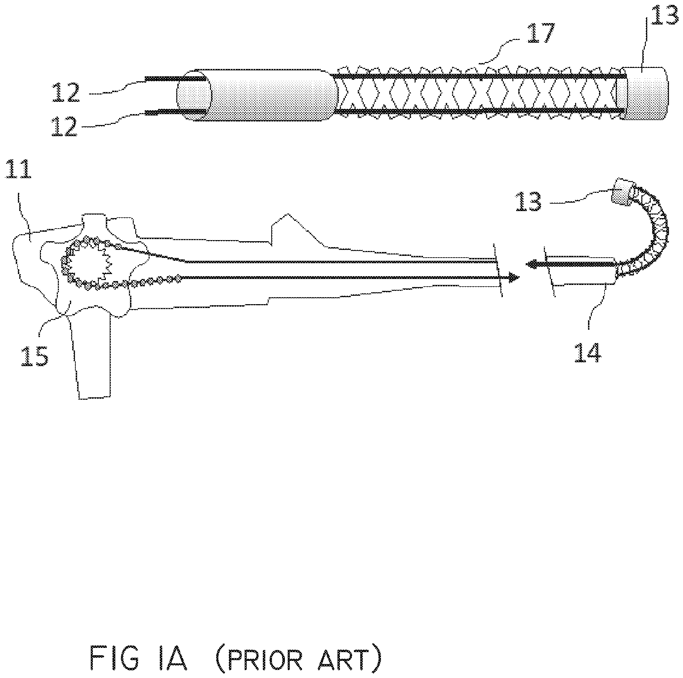

Reference is now made to *Fig. 1A**,* which illustrates schematically a prior art endoscope 11 having angulation knob 15 connected to wires 12 on opposite sides of the flexible insertion tube 14. The wires 12 pass via the insertion tube 14 via the flexible steering segment 17 and connect to the rigid tip of the endoscope 13. In such a device with a flexible steering segment 17, shown in enlarged detail above the endoscope, the position of the tip is controlled by the wires 12, in such way that the physician can manipulate the tip 13 location by turning the angulation knob 15.

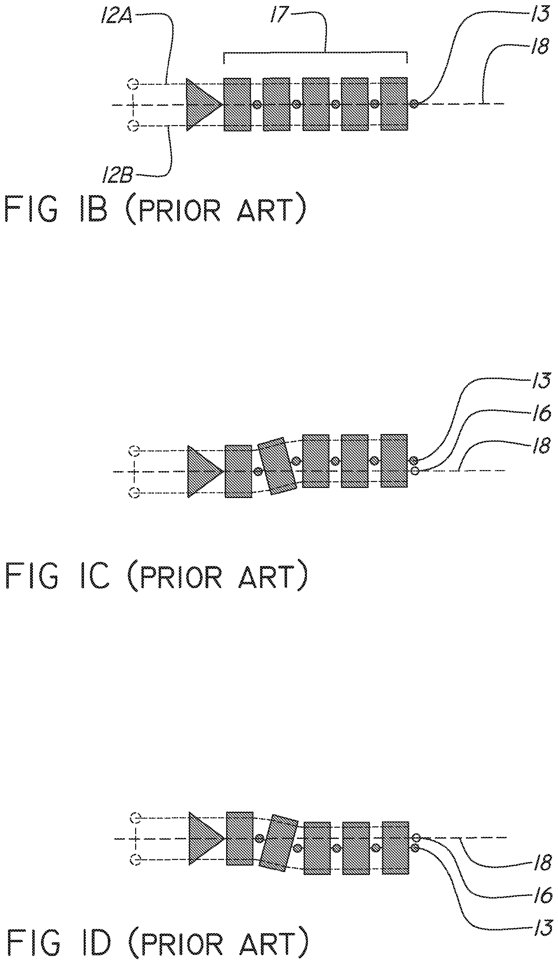

The bendable endoscope is steered by the physician relative to the three-dimensional view of the surgical field using direct vision via a camera located at the tip 13 or another real-time imaging modality. The prior art endoscope in *Fig. 1A* has two pulling wires used for steering, such that as long as the tip of the endoscope is in the intended position of the surgical field, there is no significance to which wire is pulled and the exact bending the flexible steering segment **17** or in which direction the tip is bent. This concept is illustrated schematically in *Figs. 1B-1D**,* showing the flexible steering segment **17** and the tip **13** in a straight position (*Fig. 1B*), and curved either upward (*Fig. 1C*) also known as "elbow up" in robotics or downward (*Fig. 1D*) also known as "elbow down" in robotics of a defined reference central point **16.** Line **18** is shown for reference purposes. The wires length **12** in this illustration bear no singular relationship to the position of the tip of the device **13** relative to the reference central point **16,** as explained below.

Because the length of the two wires **12A** and **12B** as shown in *Figs. 1B-1D* are the same, for equal length of either wire **12A, 12B,** the tip **13** could be located in more than one location, *i.e.,* either in the upward direction of the orientation *of* *Fig. 1C* or in the downward direction of the orientation of *Fig. ID.* This lack of precision arises because there is no directionally defined information, *i*.*e*., there is no single positional solution, contained in the form of the intermediate joints of the tip section.

In mathematical or engineering terms, this shows a steering mechanism with more than one mathematical solution, or a system with a non-injective solution. The steering mechanism shown in *Figs. 1B-1D* has four joints and two pulling cables; therefore, it is an "under-defined mechanism". If each joint had two pulling cables, thus a total of eight cables for the four joints in the illustration, or 4 "motors" for 4 joints, a single solution would be defined for the steering segment location. The significance of a single solution is that the tip of the endoscope **13** and the location of each link would be defined in two-dimensional space for any given position of the steering segment 17.

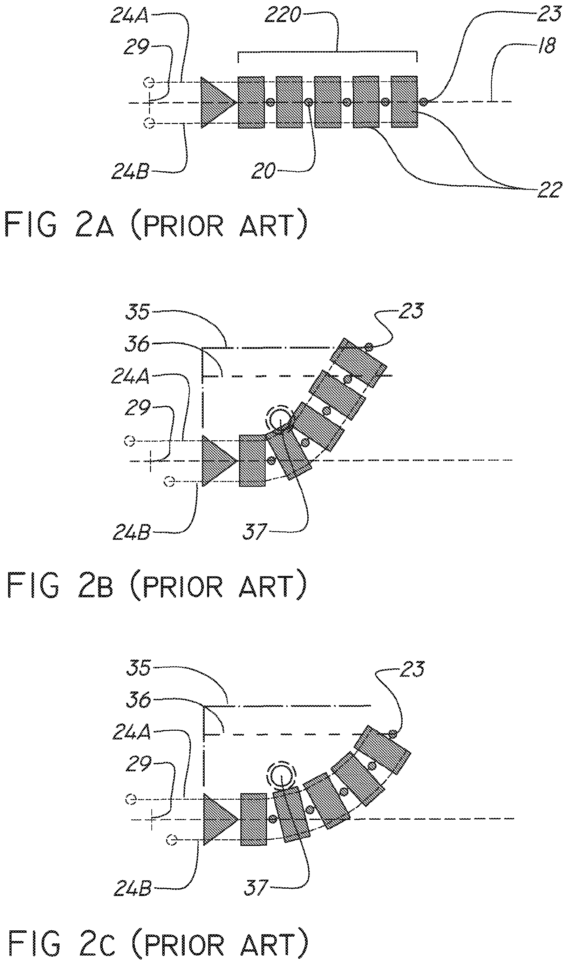

Such a steering system operates efficiently in a hollow organ such as the stomach, abdominal cavity or blood vessels where the exact location of the tip is not critical and the physician is able to adjust the tip location using real time feedback. For example, during colonoscopy or gastroscopy the physician will steer the tip of the endoscope according to live video images, or during catheterization of blood vessels the physician will use X-ray-based video images to steer the tip of the endoscope or catheter. In general, an endoscope with a steering mechanism having a non-injective solution works well when the physician acts as a "closed loop controller" based on real time images, and where the exact location of the endoscope within the body cavity is not critical, such as when the tip is used just for observation of the body cavity or lumen. However, during intracranial operations, moving the tip of the endoscope a few mm left or right may cause permanent, severe damage the patient's brain, as now illustrated in Figs. 2A to 2D below.

Reference is now made to *Fig. 2A-2C**,* illustrating the limitations of prior art endoscopy steering for intracranial procedures. In *Fig. 2A* is shown a flexible steering segment **220** in a straight position with the ends of the wires **24A, 24B** aligned with the marker **29.** Each link **22** is connected to the adjacent one with a rotary joint **20.** *Figs. 2B* and *2C* illustrate a potential problem arising from the steering of a flexible steering segment tip **23** in two situations of partially deflected positions relative to a critical intracranial object, such as a blood vessel **37.** In either partially deflected position, the relative distance between the ends of wire **24A** and marker **29,** and **24B** and marker **29** is the same; however, the steering segment links **22** in *Fig. 2B* follows a track that impinges on the blood vessel **37,** potentially causing an intracranial hemorrhage. In *Fig. 2C**,* the steering segment links **22** follow an alternate track, bypassing the blood vessel **37.** The difference in the paths *of* *Fig. 2B* and *Fig. 2C* is reflected as well in the position of the tip **23,** which reaches line **35** in *Fig. 2B**,* but to line **36** in *Fig. 2C*. Thus, even though the relative distance between the ends of the wires **24A** and **24B** and the marker **29** is the same in both drawings, from this information, neither the path of the steering segment nor the exact position of the tip can be known in a partially deflected position when using a steering mechanism lacking a single defined solution. Thus, in a clinical setting, the positional information available from the wires **24A, 24B** is insufficient to know the exact position of the steering segment tip **23** and links **22.** Such lack of precision is unacceptable in semi-solid organs such as the brain, in which the insertion of a surgical tool may damage critical functions. Thus, minimizing the track of the insertion in three-dimensional space and following a predefined path that avoids critical features are important aspects of an endoscopic device.

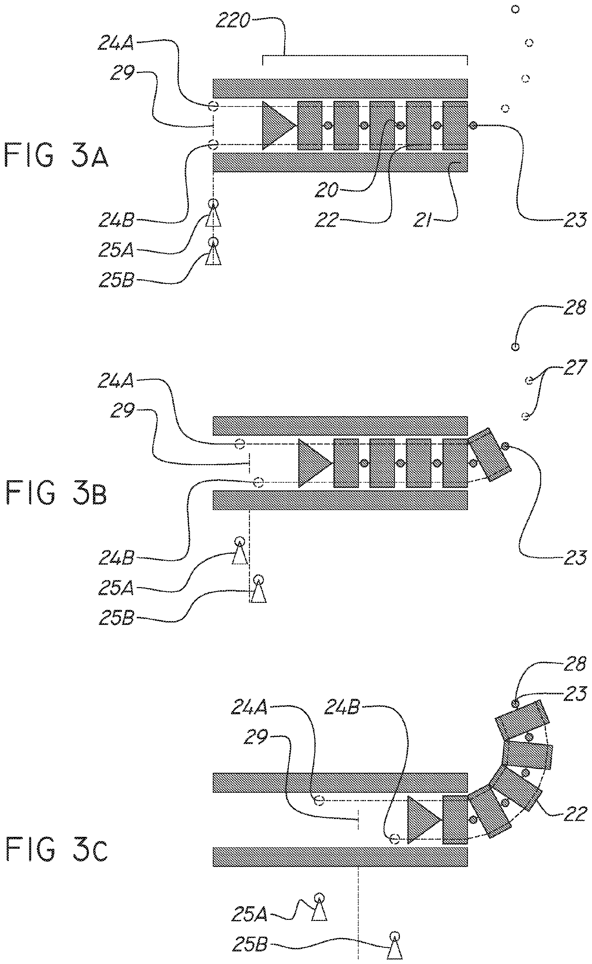

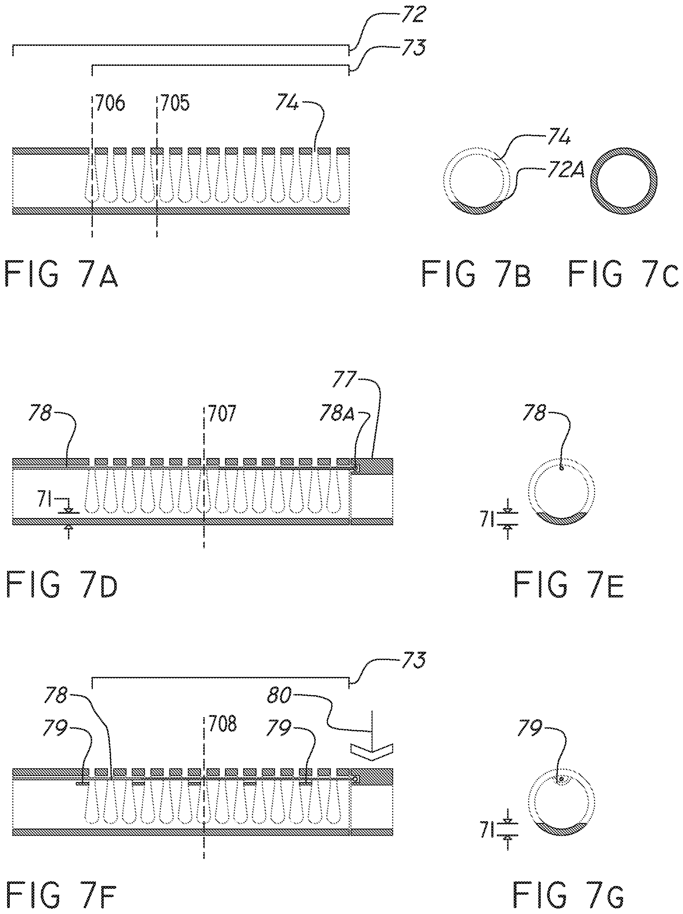

Reference is now made to *Figs. 3A-3C**,* illustrating the method of operation of the novel devices of current disclosure, which solves both of the problems mentioned above. A steerable needle or middle cannula **220** is disposed within a stiffer outer cannula **21,** both of which are shown in longitudinal section. The middle cannula **220** is composed of individual links **22** connected via two-dimensional rotary joints **20.** The links and joints of the middle cannula may be comprised of any number of suitable and conveniently available materials. They may be comprised of many parts assembled together, wherein rotary joints and rigid links are joined to a single steering segment, as shown in *Figs. 3A-3C**.* Such joints can be hinged, flexible, comprised of magnetic beads, or other solutions as will be shown and described in Figs. 7A-H below.

In *Fig. 3A**,* the proximal end position of the two pulling wires **24A, 24B** is known according to the triangular markers **25A** and **25B,** shown here not as part of the device, but to illustrate the relevant concept. Both pulling wires are aligned at opposite ends of the marker **29,** a linear indicator of the relative positions of wires **24A, 24B.** *Figs. 3B* and 3C show consecutive time points relative to *Fig. 3A* during the course of moving the middle cannula **220** out of the outer cannula **21.** Because the outer cannula **21** constrains the path of the middle cannula **220,** and each link is bent at a defined angle as it exits the confines of the outer cannula **21,** the tip of the middle cannula **23** follows a single, defined pathway, as shown by the dots **27** in *Fig. 3B**.* Thus, both the path of the middle cannula **220** and its tip **23** are known relative to a pre-defined target **28,** and the needle follows a single track through the tissue. For each linear extension of the middle cannula, the radial change can be calculated. The control of the middle cannula tip **23** may be such that each link **22** moves out of the outer cannula **21** at a radial angle of less than two degrees for example, resulting in very small steps and a high degree of precision. Further precision of movement and resolution of position beyond that allowed solely by the curvature of the middle cannula may be accomplished by integrated operation of the other motors in the system, as described below.

In *Fig. 3B**,* the tip of the middle cannula **23** moves along a curved path, conveniently guided by robotic control toward the target point **28** according to the pre-operative surgical plan, the pulling wire **24B** moves a given radial distance along the outer curve of the steerable needle. The needle **23** has a natural curve such that it will turn in an upward direction as shown in the drawing *of* *Fig. 3B**,* if the pulling wire is loose, shown by the linear movement of marker **25B** relative to marker **25A.** The angular motion of the tip **23** can be determined by the linear distance of the ends of the pulling wires **25A, 25B,** such that at any given point in time, the position of the tip **23** is known relative to the target **28.** Marker **29** provides an indication of the linear distance the entire middle cannula has moved outside the outer cannula **21.**

The robot can calculate the location of the middle cannula tip **23** based solely on (a) the travel distance of marker **29** relative to the outer cannula **21** and (b) the middle cannula **220** dimensions. The path of the middle cannula tip **23** toward the target is defined by intermediate positions **27,** as defined by the design parameters of each steerable middle cannula. At the latest time point shown in *Fig. 3C**,* the needle tip **23** has reached the target point **28** in manner such that each position of the steerable middle cannula **220** has followed the same path as the middle cannula tip **23** along the same course. This type of movement is referred to as `follow the leader mechanism', and is important for minimizing damage to normal brain tissue during insertion of a surgical tool. In this context, the `follow the leader' mechanism ensures that a single, narrow trajectory approximately the width of the needle track is the sole path through the tissue. This mechanism thus addresses a problem of tissue damage caused along the probe path.

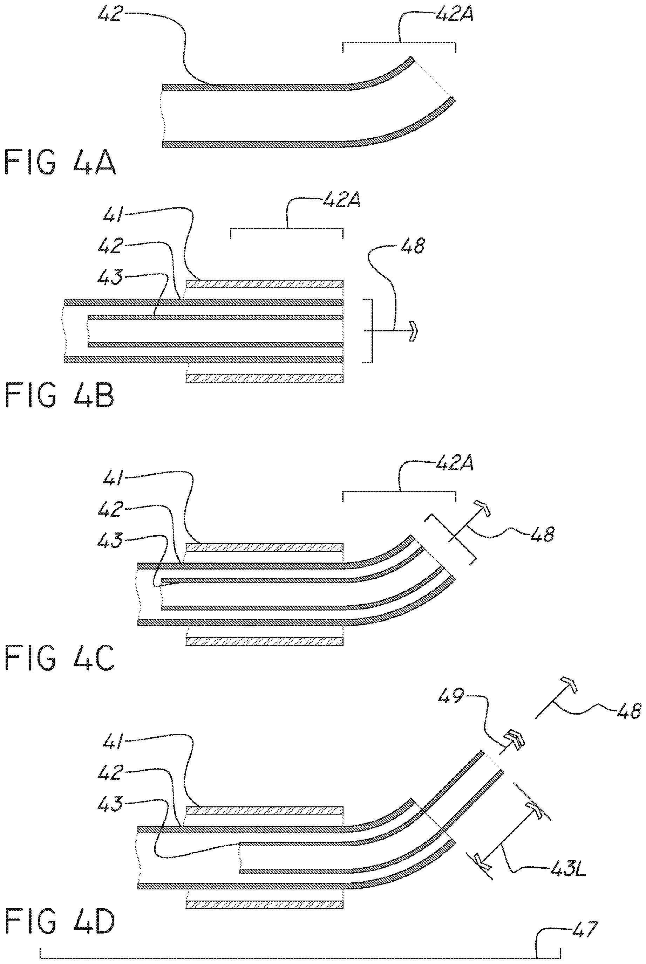

Reference is now made to *Figs. 4A-4G**,* illustrating an exemplary implementation of the present disclosure having three concentrically arranged cannulas, hereafter referred to as outer, middle and inner cannulas. A further note on nomenclature: the terms 'tube' and 'cannula' are used interchangeably to refer to any of the hollow passageways in this diagram; the innermost flexible element may comprise either a hollow tube or a solid element as described above; and `needle assembly' designates the disclosed device as shown *in* *Fig. 4D**.*

*Fig. 4A* shows the middle cannula **42** in longitudinal section. It has an intermediate stiffness such that it can hold its shape until external force applied on the middle cannula by the outer cannula straightens it, and is configured with a natural curved distal section **42A** used as a steering segment.

*Fig. 4B* shows the three concentric cannulas in longitudinal section. Outer cannula **41** is configured to be straight and rigid, without any natural curvature. As long as the middle cannula **42** is disposed completely within the outer cannula, the middle cannula maintains the straight shape enforced by the outer cannula. In the configuration shown in *Fig. 4B**,* the direction of the middle cannula, as shown by arrow **48,** is co-aligned with the outer cannula main axis. The inner cannula **43,** having the lowest stiffness, complies with the curve that the middle cannula steering segment **42A** generates as long as it is disposed within the middle cannula. However, the inner cannula **43** has self-straightening properties, such that once it extends and protrudes from the middle cannula **42,** it maintains a straight configuration. One example of how this property can be achieved are to be found in the above referenced publication US 2018/0014885 having a common inventor with the instant application or in other examples as shown below.

*Fig. 4C* shows the three concentric cannulas in longitudinal section. Here, the middle cannula **42** has extended beyond the outer cannula **41,** its direction of extension indicated by arrow **48.** Once the middle cannula **42** is moved forward and its steering segment **42A** extends and protrudes from the outer cannula **41,** then the middle cannula steering segment **42A** will curve back to its original shape as shown in *Fig. 4A**.* In such a case, the tip of the middle cannula will have a direction **48** that is not co-aligned with the axis of outer cannula **41.**

*Fig. 4D* shows that once the inner cannula **43** is moved forward and its tip extends and protrudes a length of 43L beyond the middle cannula **42,** the inner cannula will return to its original shape and straighten outside of the middle cannula. In such a case, the tip of the middle cannula will keep the direction **48** as set in *Fig. 4C**,* and the inner cannula will extend in a given trajectory **49.**

To summarize, the outer cannula **41** has the greatest stiffness, whereas the inner cannula **43** has the least stiffness. The outer and inner cannulas are inherently straight when in an unstressed situation, whereas the middle cannula **42,** having an inherent curve at its distal tip, will return to its original naturally curved shape when released from a constrained straight shape within the stiffer, outer cannula. Forward movement of middle cannula **42** relative to the outer cannula **41** generates known deflection of the direction **48** as shown in *Fig. 4C**,* whereas moving middle cannula **42** backwards into the outer cannula **41** generates a straight direction **48** of the inner cannula **43** as shown in *Fig. 4B**.* Such a tip deflection has a correlation to the forward movement of outer cannula **41** relative to middle cannula **42** (with a single solution). This design is in contrast to US 2004/0059260 to C. L. Truwit.

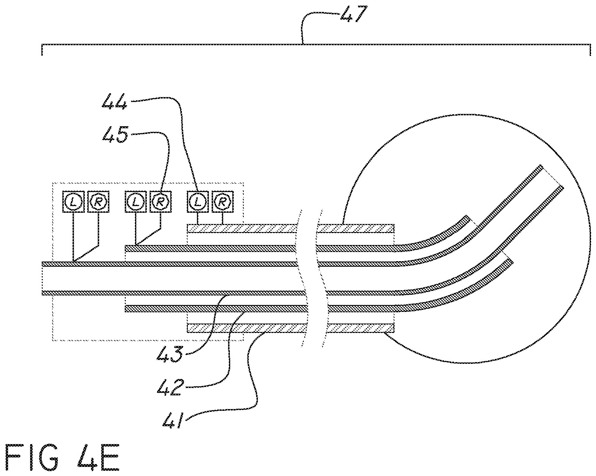

As shown in *Fig. 4E**,* linear **44** and rotational **45** motors attached to the needle assembly **47** control the extension and rotation of all three cannulas **41, 42,** and **43.** In this exemplary version of the device, a series of motors **44** causing linear extension, drives each of the three cannulas **41, 42, 43** and provides outward movement of each cannula separately relative to the needle assembly **47.** All three cannulas **41, 42,** and **43** have in addition a motor **45** providing rotational movement. As such, the needle assembly **47** can operate with six degrees of freedom, although fewer motors providing for fewer degrees of freedom are also possible.

A potential advantage of the present system is that the two outer cannulas **41, 42** provide a guide for the inner cannula **43,** such that when either the middle cannula **42** or the inner cannula **43** is extruded from within the outer cannula **41,** each assumes its natural shape. The three cannulas provide two different degrees of freedom (DOF) to the mechanism: (a) 1st DOF is an angular change - generates by the relative movement of the outer vs the middle cannula and; (b) 2nd DOF is linear change - generated by the relative movement of the middle vs the inner cannula. Also, can be described as change in direction of trajectory, and extension, thereby allowing the tip of the inner cannula to reach a two-dimensional workspace as shown in *Fig. 4D**.*

If additional DOFs are added, for example a third DOF being rotation of the outer cannula around its main axis, and a fourth DOF being linear movement of the outer cannula parallel to its main axis, the new mechanism can reach any point within a given three-dimensional workspace.

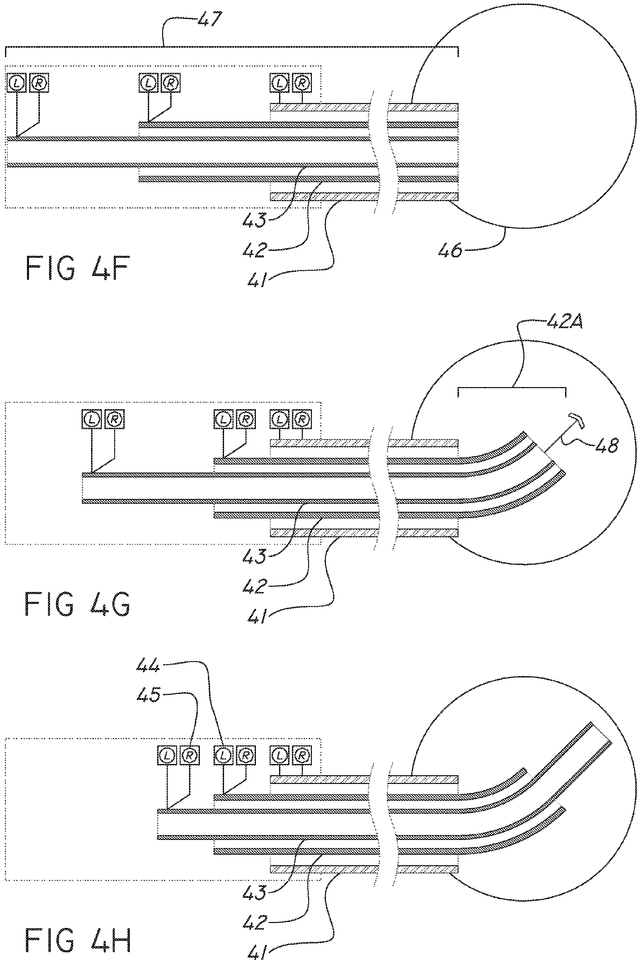

Reference is now made to *Figs. 4F-4H**,* showing sequential steps of the needle assembly **47** entering a lesion **46** within the human body to perform a procedure, such as to remove a lesion, *e.g.,* tumor, blood clot, or other abnormal or diseased tissue. In *Fig. 4F**,* the outer cannula **41** is at the periphery of the lesion, the middle cannula **42** is fully housed within the outer cannula **41,** and the inner cannula is fully housed within the middle cannula **42.**

In *Fig. 4G**,* the natural curved steering segment **42A** of the middle cannula **42** is extended beyond the end of the outer cannula **41** within the lesion. The inner cannula **43** remains completely enclosed within the middle cannula steering segment **42A,** its shape following the curvature of the middle cannula **42.** Due to its inherent curvature, the tip of the middle cannula **42** has a direction **48,** non-co-aligned with the insertion direction of the entire needle assembly shown in *Fig. 4E**.* The inner cannula is designed to self-straighten once it extends beyond the confines of the middle cannula. Thus, once the robotics system is actuated, the motor pushes the inner cannula **43** forward and the inner cannula protrudes from the middle cannula **42** in a straight manner as shown in *Fig. 4H**.* To reach other areas of the lesion at different angles, the inner cannula **43** can be extended from the middle cannula **42,** at the same time varying the extension of the middle cannula from the outer cannula **41.** It is obvious from this design, that the combination of various rotational and extensional motions of the middle cannula **42** and inner cannula **41** allows the tip of the inner cannula **43** to reach the entire three-dimensional space of the lesion by varying the operation of the six motors and the extension and rotation of the middle cannula **42** and of the inner cannula **43** from within the entire needle assembly **47.** The position of the tip of the inner cannula **43** is known to the robotic controller by measuring the linear extension of both middle and inner cannulas, and calculating the rotational angle from the extension of the middle cannula.

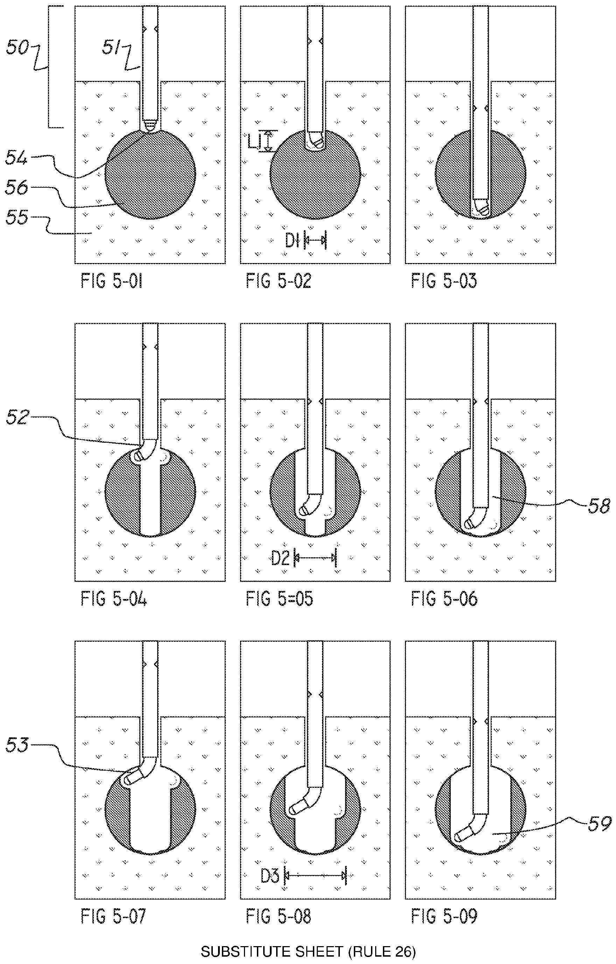

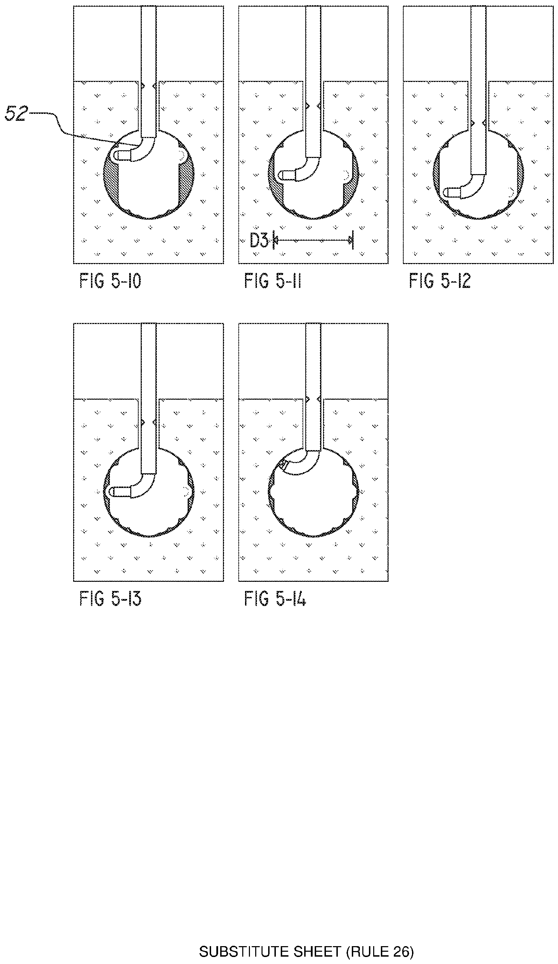

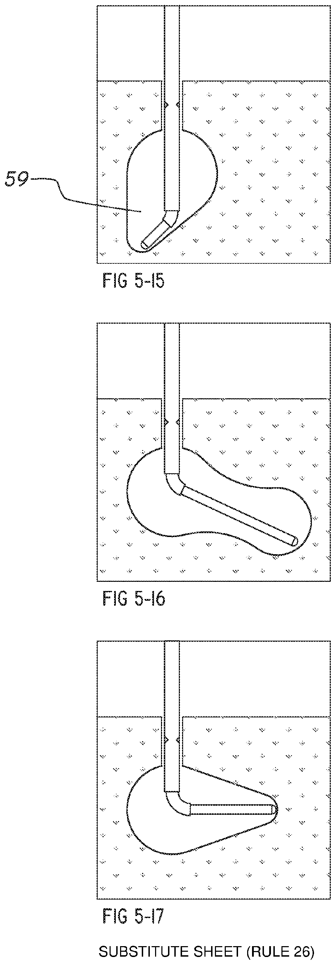

Reference is now made to Figs. 5-01 to 5-17, which illustrate schematically how the steerable needle mechanism **50** can operate within the human body to incise and remove a lesion such as a tumor or diseased brain tissue. Such a mechanism may operate as described in *Fig. 4A-*4H, with a rotating motor that can rotate all (or each) of the three cannulas around the main axis of the outer cannula **51** and a linear motor that moves the cannulas in and out of the needle assembly housing **51.** Such motors added to the steering of the inner cannula tip **54** of the mechanism allow the functional end of the device **54** to reach a three-dimensional volume of irregular size and shape, *e.g.,* a tumor, hematoma, or other diseased tissue to be excised. Additional motor(s) may be used to control the entry and exit of the entire needle assembly into the tissue to be operated.

In this example, but not limited to such manner, the device tip **54** is adapted to excavate the tumor in a sequential manner from the inside to the outside edge of the tumor, operated by a computer that programs a surgical robot to insert and rotate the needle assembly **50,** which enters and traverses the inside of a lesion in a manner allowing its tip access to sequential layers of the lesion in both vertical and horizontal directions. In various exemplary implementations, the inner element may comprise either a cannula for delivery of a substance, a surgical tool, or other device. The inner element may be used as a fine scalpel, to incise and separate the diseased tissue from the surrounding healthy tissue, in conjunction with a separately inserted suction tip to remove the excised tissue. The needle assembly may alternately be adapted to function as a suction device, wherein the inner cannula is a hollow tube and itself performs either or both of the excavation and tissue removal. Other implementations allow the inner cannula to be adapted to perform one or more of tissue ablation, excision, and removal, either simultaneously or sequentially.

Reference is now made to Figs. 5-01 to 5-14, which illustrate schematically the steps in the entire insertion and treatment process, starting from the entry point of the needle assembly **50** into the tissue **55** and lesion **56** in Fig. 5-01, and ending with removal of the full extent of the lesion in Fig. 5-14. For clarity, labeled items are not numbered on each drawing. The entire needle assembly **50** is driven under robotic control to a predetermined depth, upon reaching which, only the inner cannula tip **54** and middle cannula **52** are further extended into the lesion **56.** The inner cannula tip **54** is designed to remove brain lesions in a safe and efficient manner.

As shown in Fig. 5-02, location of tip **54** is changed by steering the middle cannula to 30 degrees. This is done by protrusion of the middle cannula **52** from the outer cannula **51.** The computer-guided steerable needle assembly **50** extends the middle cannula **52** from within the outer cannula **51,** and in doing so, the tip of the inner cannula **54** moves slightly sideway from the main axis of 50 such that now the diameter of the opening excavated by the tip **54** is D1.

The needle assembly **50** is rotated around its main axis 360 degrees, for example, while the entire mechanism moves into the lesion, such that the tip **54** removes the surrounding tissue at a diameter of D1, thereby evacuating a cylinder or tubular shape of a height referred to hereafter as layer L1, or layer height.

As shown in Fig. 5-03 the needle mechanism **50** continues to move downward or forward into the lesion while rotating, until the tip **54** reaches the lesion **56** edges, resulting in a hollow cylinder with a radius of D1 and the full height of the lesion, free of tissue, the cylinder previously holding multiple layers of tissue that have now been removed.

As shown in the subsequent phase in Fig. 5-04, the entire needle assembly 50 is raised or retracted from the depth of the lesion to the outermost layer, and the angle of the middle cannula **52** is steered to 60 degrees. This is accomplished by extending the middle cannula **52** farther from the outer cannula **51,** thereby extending the tip of the inner cannula **54** farther sideways from the main axis of the needle mechanism **50.** In Figs. 5-05 to 5-06, is shown the subsequent phase of the drilling process. The steps illustrated in Figs. 5-02 and 03 are repeated, creating an evacuated space of diameter D2. This process creates an empty cylinder **58** with a diameter of D2, such that the full height of the central part of the lesion is free of tissue.

In Figs. 5-07 to 5-09, is shown the subsequent phase of the drilling process. The position of tip **54** is changed by extending the inner cannula **53** a specific distance; the middle cannula **52** is maintained in the same place within respect to the outer cannula **51,** keeping the angle at 60 degrees as set in Fig. 5-04. The inner cannula **53** now protrudes from the middle cannula **52,** and by doing so the tip **54** of the inner cannula **53** moves farther sideways from the main axis of the needle assembly **50.** The steps in Fig. 5-02 and *5**-03,* are again repeated. By the end of this phase we have a larger cylinder **59** with a radius of D3 and the full height of the lesion free of tissue.

In Figs. 5-10 to 5-14, the process repeats, but now the angle is set to 90 degrees and eventually to 120 degrees in Fig 5-14 until the entire spherical volume of the lesion is removed. The tip of the inner cannula **54** is inserted in a linear and rotational manner that allows removal of tissue in a lesion having changing dimensions, and using finer control of the machining process to remove small residual areas of diseased tissue within the lesion.

Figs. 5-15 to 5-17 show a non-spherical lesion having a volume **59** and the manner in which the system can operate to reach the far reaches of such a volume, by extending the cannulas according to the shape of the lesion. An important feature of this flexibility of tip location is that it may be used not only for excavation of a lesion, but for reaching any known location in the brain or other solid organ, as defined by medical imaging or other localization tool.

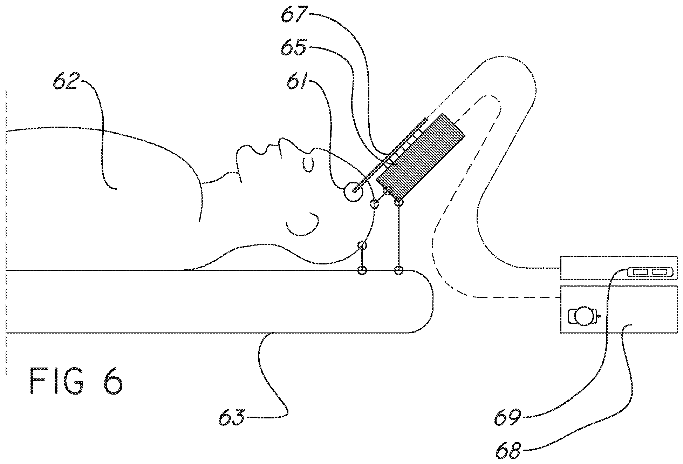

Reference is now made to *Fig.* 6, showing the manner in which the entire system operates on a patient **62** lying on an operating table **63.** The surgeon uses preoperative three-dimensional images, *e.g*., CT/MRI, to identify and map the lesion to be removed according to a pre-operative plan. This information is then fed into a computer **68,** and a controller **69** is used to program the robot to carry out the lesion resection as described above in conjunction with Fig. 5. The needle assembly **67** may then be attached, to the activating arm of a surgical robot **65** which is stably attached to the patient's skull or other bony structure. A keyhole opening **61** is made in the patient's skull, through which the needle assembly **67** is inserted into the brain to perform the programmed operation. Whereas the robot may carry out the entire operation according to the pre-operative plan, as noted above, it is also possible for the surgeon to make changes to the surgical program intraoperatively, based on intraoperative imaging or findings during the lesion resection. The surgeon may choose to use a fully manual mode, also known as "master slave robot", to navigate and operate within the brain. In this mode, the surgeon controls the movement of the cannulas manually and chooses the path and function of the inner cannula by direct vision, intraoperative monitoring, or using guidance based on preoperative imaging studies.

An example of the dimensions of a typical brain lesion that could be resected by the currently disclosed device now follow. Such dimensions are for illustrative purposes and should not be taken as limiting the scope of the device. The diameter may vary between 5 mm to 70 mm, typically falling within the range of 15-40 mm. Lesions could be spherical, as typically seen in intracranial hemorrhage having a volume ranging, *e.g.*, from 5 to 100 cc, with highly defined edges, or they could have a non-spherical shape, as typically seen in glioblastoma multiforme, with non-defined edges. The depth of such lesion could be shallow and close to the skull, requiring an overall needle length of 10 mm to 100 mm; alternately, it could be a deep lesion close to the base of the skull requiring an overall needle length of 5 mm to 150 mm.

The outer diameter of the steerable needle assembly could range between 1.5-12 mm. Depending on the outer diameter of the needle assembly, the diameters of the middle and inner cannulas are designed accordingly. In one illustrative example, the inner cannula has an outer diameter of 2.3 mm, the middle cannula has an outer diameter of 3.7 mm and the outer cannula has an outer diameter of 4.2 mm. The bending radius of such a middle cannula steering segment may be designed in the range of 2-12 mm, for example having a 3-5 mm inner bending radius.

In one illustrative example, the inner cannula has an outer diameter of 2.3 mm, the middle cannula has an outer diameter of 3.7 mm and the outer cannula has an outer diameter of 4.2 mm. The bending radius of such a middle cannula steering segment may be designed in the range of 2-12 mm, for example having a 3-5 mm inner bending radius.

Reference is now made to *Figs. 7A-7I**,* illustrating details of an exemplary design that can function as a steerable needle mechanism as described in the devices shown in *Figs. 3A-3C* and *4A-4H* above. The steerable needle mechanism is constructed to have the desired properties of both flexibility and the ability to determine the position of the tip at any given point in the operation. In the context of this application, in defining the position of a given cannula tip, 'distal' is defined as being situated away from the needle assembly, and 'proximal' is understood to mean situated closer to the needle assembly. *Fig. 7A* shows a longitudinal section of a middle cannula **72** wherein cuts or grooves **74** are made selectively in the distal part of the cannula, defined as the steering segment **73.** A cross-section taken at level **706** shows that only the lower part of the cannula **72A** remains once the cutting process **74** is completed *(**Fig. 7B**).* This remaining area **72A** is defined as a rib. A cross-section at level **705** shows that all of the cannula cross section is kept intact (*Fig. 7C*).

*Fig. 7D* shows a longitudinal section of the middle cannula **72** wherein a tip **77** is added at the leading edge of the cannula. Such a tip is connected to a pulling wire **78** via a single point **78A.** Such a pulling wire that extends along the length of the cannula and connects to a pulling motor or spring (not shown). The pulling wire may have any suitable composition, such that the material properties provide the required tensile strength and flexibility, such as a metal wire, plastic cord, or any other durable, biocompatible material. A cross-section taken at level **707** shows the location of the pulling cable **78** within the cannula *(**Fig.* 7E). In both *Figs. 7D* and *7E*, the width or cross section of the remaining material of the cannula is shown by the lines and arrows **71.**

*Fig. 7F* shows a middle cannula wherein a "U" shaped sling **79** is added in selected locations along the longitudinal length of the cannula to keep the pulling wire **78** in place. The pulling wire **78** can move freely longitudinally within the sling **79.** The cross-section taken at level **708** shows the "U" shaped sling **79** keeping the pulling wire **78** in place *(**Fig. 7G**).*

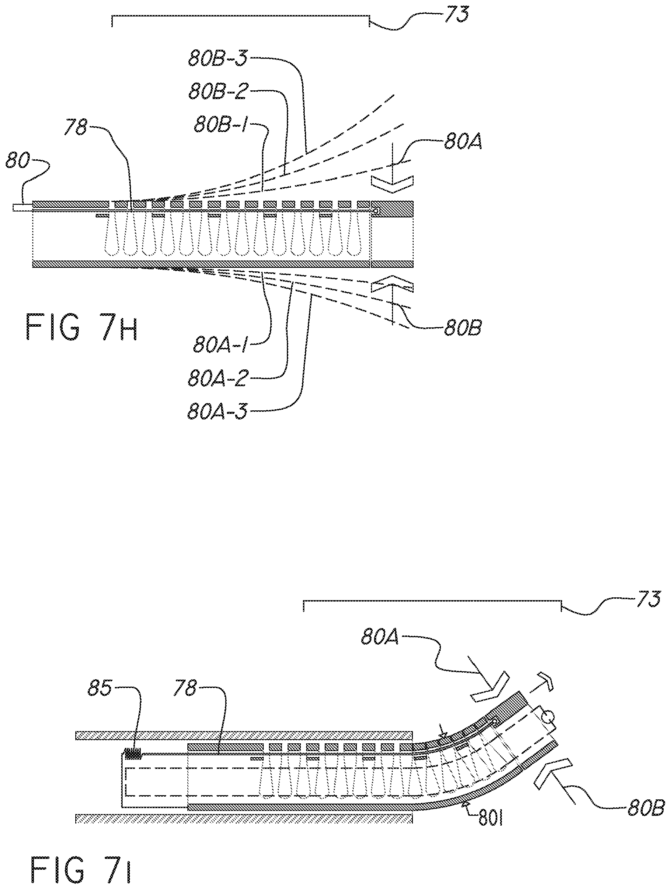

*Fig. 7H* shows a middle cannula that is easily subject to deflection due to side forces. Assuming the pulling wire **78** is firmly connect to the middle cannula at the end of the middle cannula **80,** if a side force **80A** is applied on the tip of the middle cannula, the steering segment **73** will deflect sideways as shown by dashed lines **80A-1** to **80A-3.** The deflection has a correlation to the side force power. Such deflection is (a) mostly due to the elongation of the cable **78** due to its elasticity and (b) less due to the flexibility of the middle cannula steering segment. If a side force **80B** is applied on the tip of the middle cannula the steering segment **73** will deflect sideways, as shown by dashed line **80B-1** to **80B-3.** The deflection has linear correlation to the side force power. Such deflection is due to the flexibility of the middle cannula steering segment **73;** the cable 78 will not contribute or reduce since it has no active contribution.

Reference is now made to Fig. 7I, showing a middle cannula with the capacity to retain its shape under side forces. Assuming the pulling wire **78** is connected to the middle cannula at the end of the middle cannula **80** using a pre-stressed spring **85,** if a side force **80A** is applied on the tip of the middle cannula the steering segment **73,** force will be applied on the cable **78.** If the cable will elongate due to its elasticity, the spring **85** will compensate and the steering segment will keep its closed shape. Once the side force **80A** will overcome the spring **85** force, the steering segment will start deflecting. If a side force **80B** is applied on the tip of the middle cannula the steering segment 73 will not deflect sideways since the gaps in the steering segment are closed.

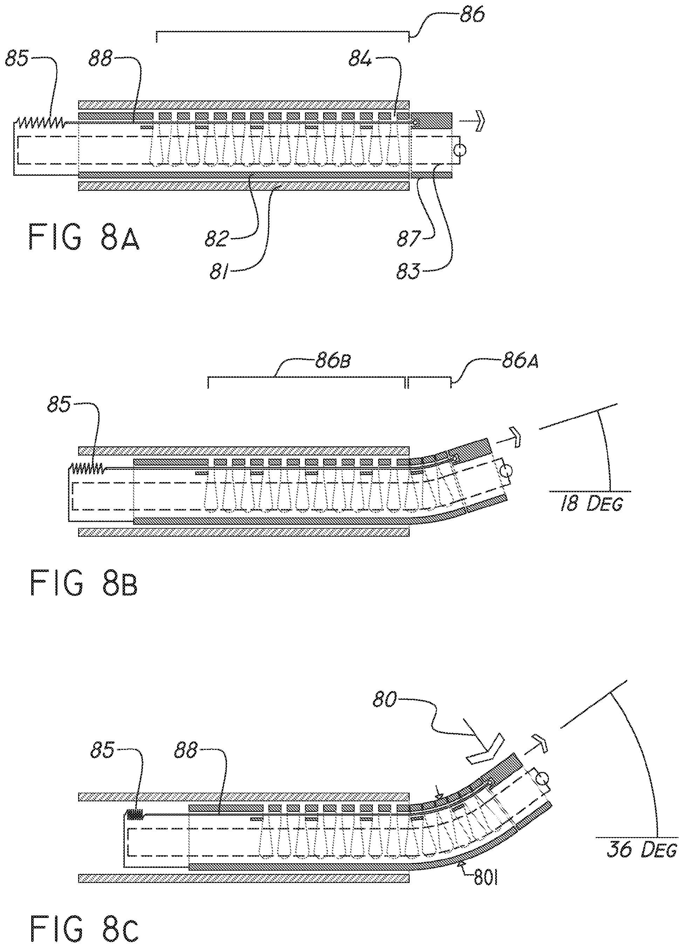

Reference is now made to *Fig. 8A**,* showing a middle cannula **82** as described above, housed within an outer cannula **81,** the outer cannula being stiffer than the middle cannula. A spring **85** is located at the proximal end of the middle cannula **82.** One end of the spring is connected to the proximal end of the wire **88** and the other end is anchored to the proximal tail of the middle cannula. The spring **85** is loaded and a consequential pulling force is applied on the pulling cable **88** and hence onto the tip **87.** However, due to the greater stiffness of the outer cannula **81,** the middle cannula is kept straight.

Reference is now made to *Fig. 8B**,* showing the result of the middle cannula **82** being pushed distally and forward relative to the outer cannula **81.** Now, due to (i) the force applied on the tip by the cable and, (ii) the cuts **84** in the wall of the middle cannula **82,** the segment of the cannula steering segment **86A** curves upwards in the direction shown in *Fig. 8B**.* The first three cuts or grooves in the situation shown *in* *Fig. 8B**,* are now outside the confines of the outer cannula **81,** which no longer enfolds the front part of the cannula steering segment **86.** The open part of the cannula steering segment **86A** is free to change shape and curve in the upwards direction in the drawing perspective of *Fig. 8B**.* The cannula steering segment **86B** is housed within the outer cannula **81,** and does not change its direction. In the example shown in *Figs. 8A* to *8C**,* the longitudinal length of each cut in the middle cannula **84** is such as to cause a deflection of exactly six degrees of the tip of the device, when the gaps due to the cuts are completely closed from the bending of the cannula; thus, extension of three cuts outside of the outer cannula, results in a deflection angle of 18 degrees from the main axis. Thus, it is possible to plan the tip deflection angle from the main axis according to the linear movement of the middle cannula **82** within the outer cannula **81.** During this process the spring **85** changes its length a little but still keeps the wire under stress. In this example, each segment generates exactly six degrees of deflection, but any other configuration may apply with different angles.

In *Fig. 8C* is illustrated a further extension of the middle cannula **82** relative to the outer cannula **81,** in which the tip **87** has moved outward to such an extent that six cuts are in the open, corresponding to an angle of 36 degrees as shown. The spring **85** may apply many times more force, e.g., 2x or 10x greater than what is needed, to keep the links closed as the middle cannula **82** extends outward. The greater force ensures that the tip of the middle cannula maintains a stiffness minimizing movement due to strain from passage through the tissue.

In *Figs. 8A* to *8C**,* the middle cannula **82** is held under tension by the spring **85,** as a result of which, the spaces constituting the slots on the inside curve of the curved cannula section, are closed up until they touch and abut against each other, to generate effectively a "solid wall" on the inside of the curve. If a side force **80** is now applied to the tip, as shown in *Fig. 8C**,* the middle cannula will behave as a solid annulus of material. Because of the strength of the tensioned wire providing tension within, the solid-walled, curved cannula will be significantly more resistant to bending than the slotted cannula. In its curved, tensioned form, the cannula behaves much more resiliently to side forces than in its slotted form. Expressed quantitatively, the resistance to bending is now defined by the second moment of area of the dimensioned shape **801** in *Fig. 8C**,* which corresponds to the complete cannula diameter, and is significantly larger than 71 that shown in *Fig. 7E**,* resulting in an effectively stiff cannula. The importance of this feature is that the position of the tip of the cannula can be known at any given point of the operation, since the tip of the middle cannula **82** will resist deflection caused by changes in tissue density or other forces. The tip position is thus significantly more accurately known as a result of lateral force deflection rested by the tensioned wire.

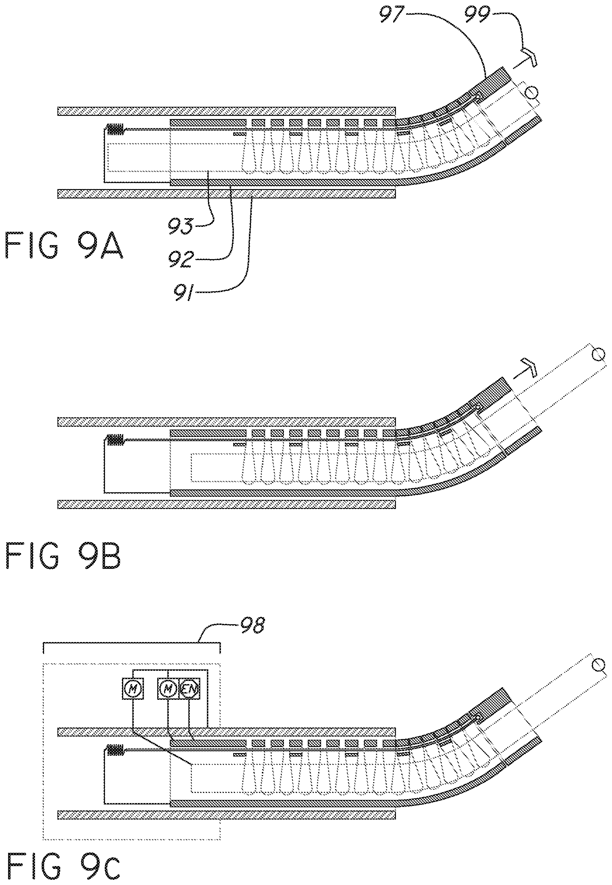

Reference is now made to *Figs. 9A**,* showing extension of the inner cannula **93** from the middle cannula **92** in a fixed position relative to the outer cannula **91,** with the tip **97** of the middle cannula partially extended outward. *InFig. 9B,* the inner cannula **93** is pushed forward and extends from the middle cannula steering segment. It extends in a straight line, keeping the same trajectory **99** at all times but changing the distance the inner needle is extended. *Fig. 9C* shows a set of motors **98** for controlling linear and rotational movement of the inner and middle cannulas.

In *Figs. 4A**-**9C**,* the outer cannula is comprised of a stiff material such as stainless steel. The middle cannula may be comprised of a round rod base spring, a flat rod base spring, a stainless steel pipe, a nitinol pipe, or a hollow structural section tube like Rectangular Hollow Section (RHS) or other materials. The inner cannula is comprised of a material having greater flexibility than either of the middle or outer cannulas. The inner cannula has the further requirement to have flexibility and the ability to return to its natural shape when not under tension. The process of pulling the guide wire may be either manual or by a motor. Furthermore, the material of which the three cannulas are comprised must be biocompatible and sterilizable.

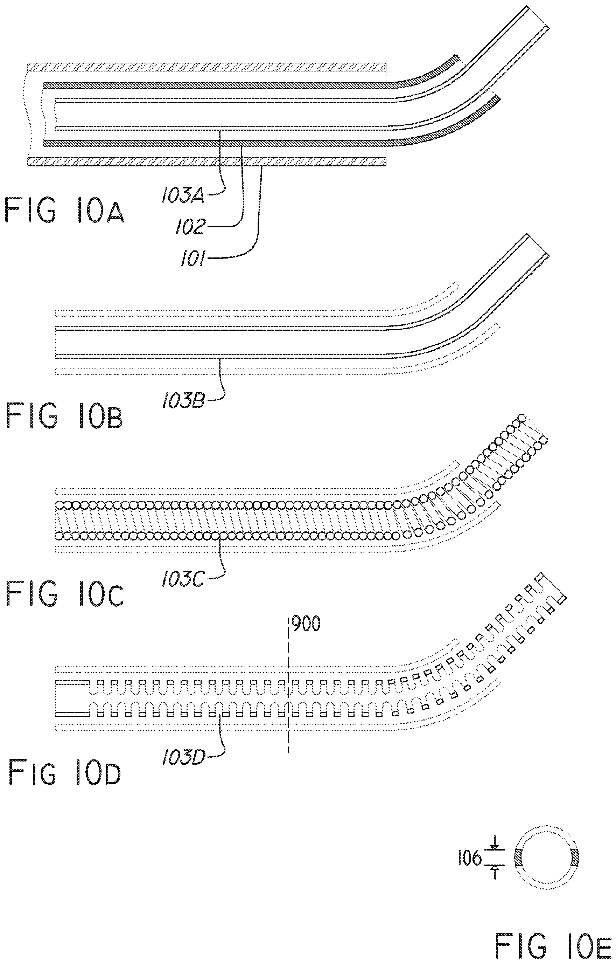

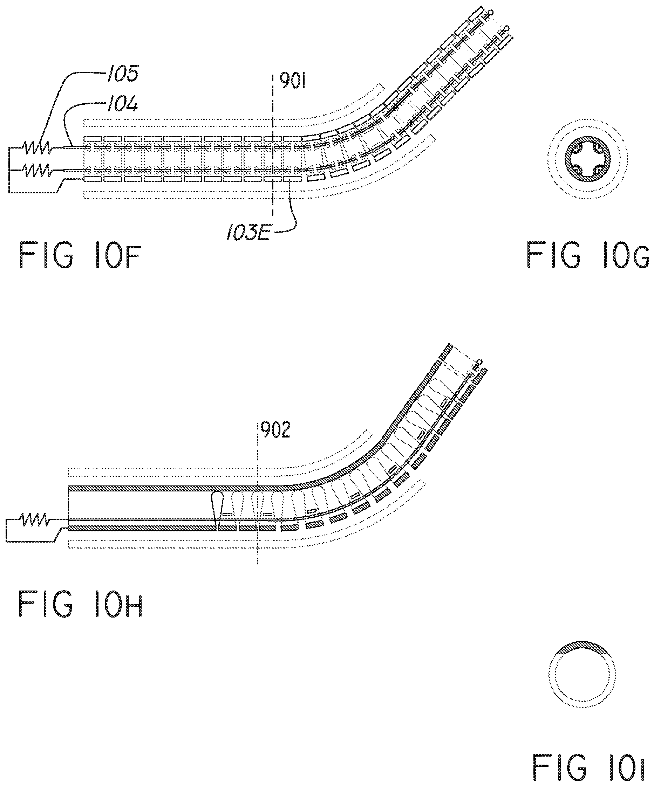

Reference is now made to *Fig. 10A-10I**,* showing various solutions for obtaining an inner cannula with suitably flexible characteristics. *Fig. 10A* illustrates the needle assembly with outer **101,** middle **102,** and inner **103A** cannulas as shown in *Fig. 4D**.* *Figs. 10B-10D*, 10F and 10H illustrate five different means of obtaining an inner cannula with suitable flexibility. *Fig. 10B* shows a needle **103B** comprised of a solid flexible material such as stainless steel or nitinol. Nitinol has two special qualities: a memory shape effect, such that bending up to a specific tension will allow the item to return to its original shape; and superelasticity, meaning that items made of this alloy have a much greater flexibility than ordinary metals. Both of these qualities make shape memory alloys such as nitinol an ideal material of which to make the flexible needle in the shape of a narrow and thin-walled tube. In *Fig. 10C**,* another possible solution for the flexible and shape-retaining cannula is shown. In this case, the cannula **103C** is comprised of a tightly coiled spring, shown in longitudinal section. In *Fig. 10D**,* the cannula has slots, as shown for the middle cannula **72** in *Fig.* 7; however, in *Fig. 10D**,* the slots in this inner cannula are on the upper and lower surfaces, as more clearly shown in cross-section **900** in *Fig. 10E**.* These slots, grooves, or cuts weaken the needle and give it a greater flexibility than the same needle comprised of the same material without the slots. Different metals or other materials could be used, and the size and width of the slots would be empirically determined to result in the desired degree of stiffness and flexibility. *Fig. 10F* shows yet another means of obtaining an inner cannula with the requisite flexibility. In this implementation, the cannula **103E** is comprised of circular links, held together by **4** pulling cables **104** preloaded by four springs **105.** This exemplary implementation is shown in cross-section **901** in *Fig. 10G. Fig. 10H* shows an inner cannula with a notched lower surface having a cable connected via a loaded spring to tension the inner cannula, shown in cross-section **902** in *Fig. 10I**.* The inner cannula will self-straighten once outside the middle cannula.

These various solutions shown in *Figs. 10A-10I* for providing a flexible, self-straightening inner cannula may be further adapted to allow the inner cannula to function during operation as a suction device, as an ablation tool, may be fitted with a microscopic camera, or may carry any other surgically relevant operating element.

It is to be understood that, whereas the majority of this disclosure discusses the application of the current flexible needle assembly to brain tissue, either tumors or thrombi from intracerebral hemorrhages, variations of the invention are possible and desirable. For example, the same system may be used to remove tumors or other lesions in other types of tissue. The design has potential advantages that allow the flexible inner cannula to access narrow joint spaces and remove damaged or torn cartilage, for example. The same stiff outer cannula could be used to house any variety of surgical tools, such as a cutter, suction, cautery, drill, curette, camera, and others.

### General

As used herein with reference to quantity or value, the term "about" means "within ±10% of'.

The terms "comprises", "comprising", "includes", "including", "having" and their conjugates mean: "including but not limited to".

The term "consisting of" means: "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example" or "exemplary" is not necessarily to be construed as preferred or advantageous over other embodiments and/or to exclude the incorporation of features from other embodiments.

The word "optionally" is used herein to mean "is provided in some examples and not provided in other examples". Any particular example of the present disclosure may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Throughout this application, examples and embodiments may be presented with reference to a range format. It should be understood that the description in range format is merely for convenience and brevity and should not be construed as an inflexible limitation on the scope of descriptions of the present disclosure. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", *etc.;* as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Although descriptions of the present disclosure are provided in conjunction with specific examples and embodiments, it is evident that many alternatives, modifications and variations will be apparent to those skilled in the art.

Citation or identification of any reference in this application shall not be construed as an admission that such reference is available as prior art to the present disclosure. To the extent that section headings are used, they should not be construed as necessarily limiting.

It is appreciated that certain features which are, for clarity, described in the present disclosure in the context of separate examples and embodiments, may also be provided in combination in a single example . Conversely, various features, which are, for brevity, described in the context of a single example, may also be provided separately or in any suitable subcombination or as suitable in any other described example and embodiment of the present disclosure. Certain features described in the context of various examples and embodiments are not to be considered essential features of those examples and embodiments, unless the example is inoperative without those elements.

## Claims

1. A needle assembly for guided extension through tissue in a surgical procedure, comprising:
a stiff, straight outer first cannula (81), terminating distally in a distal-facing aperture;
a second cannula (72) adapted to be passed through and extended out of the distal-facing aperture of said stiff straight outer first cannula (81), said second cannula (72) being configured to bend to define a known angle of curvature after extending from the distal-facing aperture; and
a self-straightening inner element (93) disposed within said second cannula (72), said self-straightening inner element (93) having a flexibility such that it passes through said second cannula (72) even when said second cannula (72) is bent, but adopts its straight form after exiting said second cannula (72);
wherein, relative to the first cannula (81), the known angle of curvature of the second cannula (72) and the straight form of the self-straightening inner element (93) together allow a tip position of the self-straightening inner element (93) to be known from the linear extension of the second cannula (72) and the self-straightening inner element (93);
**characterised in that**
the second cannula (72) has a slotted wall, slotted by a plurality of lateral slots (74) along at least part of the slotted wall, said slots (74) extending circumferentially across a majority of the wall's circumference;
a wire (78) is disposed within the second cannula (72), along the slotted wall of the second cannula (72), and attached to a distal end region of said second cannula (72);
wherein the wire (78) is under tension to bend said second cannula (72), when the slotted wall is extended from the straight outer first cannula (81), until adjacent sides of said slots (74) in said slotted wall abut each other, said abutting of the sides of the slots (74) defining the known angle of curvature of said second cannula (72); and
said wire (78) is tensioned by means of a spring (85) connected between said wire (78) and an anchor point on a proximal point of said second cannula (72).

2. The needle assembly of claim 1, wherein the spring (85) preloads the wire (78) so that the second cannula (72) bends upon being pushed distally relative to the first cannula (81).

3. A needle assembly according to claim 1, further comprising retaining devices positioned at intermediate locations along said slotted wall, adapted to thereby maintain said wire (78) in position along said slotted wall.

4. The needle assembly of claim 1, wherein ribs (72A) divide the slots (74) around their circumference.

5. The needle assembly of claim 1, wherein the slots (74) extend around their circumference in divided sections.

6. The needle assembly of claim 1, wherein, around the majority of the wall's circumference, the slots (74) are on surfaces upper and lower to ribs comprising areas of the wall remaining after the slots (74) are cut.

7. The needle assembly of claim 1, wherein, while the second cannula (72) is extended out of the first cannula (81) and the wire (78) is under tension, the second cannula (72) is configured to perform rotational motion around a main longitudinal axis of the first cannula (81) by movement relative to the first cannula (81).

8. The needle assembly of claim 1, wherein, while the second cannula (72) is extended out of the first cannula (81) and the wire (78) is under tension, the second cannula (72) extends away from a longitudinal axis of the end of the first cannula (81).

9. A needle assembly according to any one of claims 1-8, wherein any of said first cannula (81), said second cannula (72), and said inner element (93) are robotically controlled to provide at least one of extension and rotational motion.

10. A needle assembly according to any one of claims 1-8, wherein said needle assembly is robotically controlled according to a pre-surgical plan, such that a tip of said inner element (93) can reach a target region.

11. A needle assembly according to any one of claims 1-8, wherein extension of said second cannula (72) through said tissue is adapted to trace a track set by the known angle of curvature of said second cannula (72), such that damage to said tissue is minimized.

12. A needle assembly according to claim 1, wherein the known angle of curvature is dependent on the ratio between the width of said slots (74) and the width of the wall between said slots (74).

13. A needle assembly according to any one of claims 1-8, wherein the position of said distal end of said second cannula (72) is determined by the extent of said extension of said second cannula (72) out of said first cannula (81).

14. The needle assembly according to any of claims 1-13, wherein spring (85) also tensions wire (78) while the second cannula (72) is straight and contained within the first cannula (81).

## Patentansprüche

1. Nadelanordnung zur geführten Ausdehnung durch Gewebe bei einem chirurgischen Eingriff, bestehend aus:
einer steife, gerade äußere erste Kanüle (81), die distal in einer zum distalen Ende weisende Öffnung endet;
einer zweite Kanüle (72), die so angepasst ist, dass sie durch die nach distal weisende Öffnung der steifen, geraden äußeren ersten Kanüle (81) hindurchgeführt und aus dieser herausgefahren werden kann, wobei die zweite Kanüle (72) so gestaltet ist, dass sie sich biegt, um einen bekannten Krümmungswinkel zu definieren, nachdem sie aus der zum distalen Ende weisende Öffnung herausgefahren wurde; und
einem selbstausrichtendes Innenelement (93), das innerhalb der zweiten Kanüle (72) angeordnet ist, wobei das selbstausrichtende Innenelement (93) eine solche Flexibilität aufweist, dass es durch die zweite Kanüle (72) hindurchgeht, selbst wenn die zweite Kanüle (72) gebogen ist, aber seine gerade Form annimmt, nachdem es aus der zweiten Kanüle (72) austritt;
wobei in Bezug auf die erste Kanüle (81) der bekannte Krümmungswinkel der zweiten Kanüle (72) und die gerade Form des selbstaufrichtenden Innenelements (93) zusammen eine Spitzenposition des selbstaufrichtenden Innenelements (93) aus der linearen Ausdehnung der zweiten Kanüle (72) und des selbstaufrichtenden Innenelements (93) bekannt sein lassen;
**dadurch gekennzeichnet, dass**
die zweite Kanüle (72) eine geschlitzte Wand aufweist, die durch eine Vielzahl von seitlichen Schlitzen (74) entlang mindestens eines Teils der geschlitzten Wand geschlitzt ist, wobei sich die Schlitze (74) in Umfangsrichtung über einen Großteil des Wandumfangs erstrecken;
ein Draht (78) ist innerhalb der zweiten Kanüle (72) entlang der geschlitzten Wand der zweiten Kanüle (72) angeordnet und an einem distalen Endbereich der zweiten Kanüle (72) befestigt;
wobei der Draht (78) unter Spannung steht, um die zweite Kanüle (72) zu biegen, wenn die geschlitzte Wand von der geraden äußeren ersten Kanüle (81) ausgefahren wird, bis benachbarte Seiten der Schlitze (74) in der geschlitzten Wand aneinanderstoßen, wobei das Anstoßen der Seiten der Schlitze (74) den bekannten Krümmungswinkel der zweiten Kanüle (72) definiert; und
der Draht (78) mittels einer Feder (85) gespannt wird, die zwischen dem Draht (78) und einem Verankerungspunkt an einem proximalen Punkt der zweiten Kanüle (72) verbunden ist.

2. Nadelanordnung nach Anspruch 1, wobei die Feder (85) den Draht (78) vorspannt so dass sich die zweite Kanüle (72) biegt, wenn sie relativ zur ersten Kanüle (81) nach distal geschoben wird.

3. Nadelanordnung nach Anspruch 1, die außerdem Haltevorrichtungen umfasst die an Zwischenpositionen entlang der geschlitzten Wand positioniert sind, die so angepasst sind, dass sie dadurch den Draht (78) entlang der geschlitzten Wand in Position halten.

4. Nadelanordnung nach Anspruch 1, bei der Rippen (72A) die Schlitze (74) unterteilen um ihren Umfang herum.

5. Nadelanordnung nach Anspruch 1, wobei sich die Schlitze (74) in geteilten Abschnitten um ihren Umfang erstrecken.

6. Nadelanordnung nach Anspruch 1, wobei sich die Schlitze (74) um den Großteil des Wandumfangs herum auf Flächen befinden, die sich oberhalb und unterhalb von Rippen befinden, die Bereiche der Wand umfassen, die nach dem Schneiden der Schlitze (74) verbleiben.

7. Nadelanordnung nach Anspruch 1, wobei, während die zweite Kanüle (72) aus der ersten Kanüle (81) ausgefahren ist und der Draht (78) unter Spannung steht, ist die zweite Kanüle (72) so konfiguriert, dass sie eine Drehbewegung um eine Hauptlängsachse der ersten Kanüle (81) durch Bewegung relativ zur ersten Kanüle (81) ausführt.

8. Nadelanordnung nach Anspruch 1, wobei, während die zweite Kanüle (72) aus der ersten Kanüle (81) ausgefahren ist und der Draht (78) unter Spannung steht, erstreckt sich die zweite Kanüle (72) von einer Längsachse des Endes der ersten Kanüle (81) weg.

9. Nadelanordnung nach einem der Ansprüche 1-8, wobei eines der folgenden Elemente, nämlich die erste Kanüle (81), die zweite Kanüle (72) und das Innenelement (93), robotergesteuert sind, um zumindest eine Ausdehnungs- oder Rotationsbewegung zu ermöglichen.

10. Nadelanordnung nach einem der Ansprüche 1-8, wobei die Nadelanordnung gemäß einem präoperativen Plan robotergesteuert ist, so dass eine Spitze des inneren Elements (93) einen Zielbereich erreichen kann.

11. Nadelanordnung nach einem der Ansprüche 1-8, wobei die Ausdehnung der zweiten Kanüle (72) durch das Gewebe so angepasst ist, dass sie einer Spur folgt, die durch den bekannten Krümmungswinkel der zweiten Kanüle (72) festgelegt ist, so dass eine Beschädigung des Gewebes minimiert wird.

12. Nadelanordnung nach Anspruch 1, wobei der bekannte Krümmungswinkel vom Verhältnis zwischen der Breite der Schlitze (74) und der Breite der Wand zwischen den Schlitzen (74) abhängt.

13. Nadelanordnung nach einem der Ansprüche 1-8, wobei die Position der des distalen Endes der zweiten Kanüle (72) durch das Ausmaß der Ausdehnung der zweiten Kanüle (72) aus der ersten Kanüle (81) heraus bestimmt wird.

14. Nadelanordnung nach einem der Ansprüche 1-13, wobei die Feder (85) auch den Draht (78) spannt, während die zweite Kanüle (72) gerade ist und sich in der ersten Kanüle (81) befindet.

## Revendications

1. Ensemble aiguille pour une extension guidée à travers le tissu lors d'une procédure chirurgicale, comprenant :
une première canule extérieure rigide et droite (81), se terminant distalement par une ouverture faisant face à la partie distale ;
une deuxième canule (72) adaptée pour être passée à travers et étendue hors de l'ouverture faisant face à la partie distale de ladite première canule extérieure rigide et droite (81), ladite deuxième canule (72) étant configurée pour se plier afin de définir un angle de courbure connu après s'être étendue à partir de l'ouverture faisant face à la partie distale ; et
un élément intérieur auto-redressable (93) disposé à l'intérieur de ladite deuxième canule (72), ledit élément intérieur auto-redressable (93) ayant une flexibilité telle qu'il passe à travers ladite deuxième canule (72) même lorsque ladite deuxième canule (72) est courbée, mais adopte sa forme droite après être sorti de ladite deuxième canule (72) ;
dans lequel, par rapport à la première canule (81), l'angle de courbure connu de la deuxième canule (72) et la forme droite de l'élément intérieur auto-redressable (93) permettent ensemble une position de pointe de l'élément intérieur auto-redressable (93) d'être connue à partir de l'extension linéaire de la deuxième canule (72) et de l'élément intérieur auto-redressable (93) ;
**caractérisée par le fait que**
la deuxième canule (72) a une paroi fendue, fendue par une pluralité de fentes latérales (74) le long d'au moins une partie de la paroi fendue, lesdites fentes (74) s'étendant circonférentiellement sur la majorité de la circonférence de la paroi ;
un fil (78) est disposé à l'intérieur de la deuxième canule (72), le long de la paroi fendue de la deuxième canule (72), et attaché à une région d'extrémité distale de ladite deuxième canule (72) ;
dans lequel le fil (78) est sous tension pour plier ladite deuxième canule (72) lorsque la paroi fendue est étendue à partir de la première canule extérieure (81) droite, jusqu'à ce que les côtés adjacents desdites fentes (74) dans ladite paroi fendue soient en butée l'un contre l'autre, ladite butée des côtés des fentes (74) définissant l'angle de courbure connu de ladite deuxième canule (72) ; et
ledit fil (78) est tendu au moyen d'un ressort (85) connecté entre ledit fil (78) et un point d'ancrage sur un point proximal de ladite deuxième canule (72).

2. Ensemble aiguille de la revendication 1, dans lequel le ressort (85) précharge le fil (78) de sorte que la deuxième canule (72) se plie lorsqu'elle est poussée distalement par rapport à la première canule (81).

3. Ensemble aiguille selon la revendication 1, comprenant en outre des dispositifs de retenue positionnés à des endroits intermédiaires le long de ladite paroi fendue, adaptés pour maintenir ainsi ledit fil (78) en position le long de ladite paroi fendue.

4. Ensemble aiguille de la revendication 1, dans lequel des nervures (72A) divisent les fentes (74) autour de leur circonférence.

5. Ensemble aiguille de la revendication 1, dans lequel les fentes (74) s'étendent autour de leur circonférence en sections divisées.

6. Ensemble aiguille de la revendication 1, dans lequel, autour de la majorité de la circonférence de la paroi, les fentes (74) se trouvent sur des surfaces supérieures et inférieures par rapport aux nervures comprenant des zones de la paroi qui restent après la découpe des fentes (74).

7. Ensemble aiguille de la revendication 1, dans lequel, alors que la deuxième canule (72) est étendue hors de la première canule (81) et que le fil (78) est sous tension, la deuxième canule (72) est configurée pour effectuer un mouvement de rotation autour d'un axe longitudinal principal de la première canule (81) par un mouvement par rapport à la première canule (81).

8. Ensemble aiguille de la revendication 1, dans lequel, alors que la deuxième canule (72) est étendue hors de la première canule (81) et que le fil (78) est sous tension, la deuxième canule (72) s'étend à l'écart d'un axe longitudinal de l'extrémité de la première canule (81).

9. Ensemble aiguille selon l'une quelconque des revendications 1 à 8, dans lequel l'une quelconque parmi ladite première canule (81), ladite deuxième canule (72) et ledit élément intérieur (93) est commandée robotiquement pour fournir au moins l'un des mouvements d'extension et de rotation.

10. Ensemble aiguille selon l'une quelconque des revendications 1 à 8, dans lequel ledit ensemble aiguille est commandé robotiquement selon un plan pré-chirurgical, de sorte qu'une pointe dudit élément intérieur (93) puisse atteindre une région cible.

11. Ensemble aiguille selon l'une quelconque des revendications 1 à 8, dans lequel l'extension de ladite deuxième canule (72) à travers ledit tissu est adaptée pour tracer une voie définie par l'angle de courbure connu de ladite deuxième canule (72), de manière à minimiser les dommages causés audit tissu.

12. Ensemble aiguille selon la revendication 1, dans lequel l'angle de courbure connu dépend du rapport entre la largeur desdites fentes (74) et la largeur de la paroi entre lesdites fentes (74).

13. Ensemble aiguille selon l'une quelconque des revendications 1 à 8, dans lequel la position de ladite extrémité distale de ladite deuxième canule (72) est déterminée par l'étendue de ladite extension de ladite deuxième canule (72) hors de ladite première canule (81).

14. Ensemble aiguille selon l'une quelconque des revendications 1 à 13, dans lequel le ressort (85) tend également le fil (78) alors que la deuxième canule (72) est droite et contenue dans la première canule (81).
